# EUROPEAN PATENT SPECIFICATION

(11) **EP 4 232 436 B1**
(45) Date of publication and mention of the grant of the patent: **21.10.2026**
(21) Application number: 21806910.2
(22) Date of filing: 20.10.2021
(51) Int. Cl.: C07D 237/16, C07D 237/32, A61K 31/50, A61K 31/502, A61P 1/00, A61P 1/16, A61P 1/18, A61P 3/00, A61P 3/04, A61P 3/06, A61P 3/10, A61P 5/00, A61P 5/14, A61P 9/00, A61P 9/10, A61P 29/00, A61P 35/00

(54) **PROCESS FOR THE PREPARATION OF 6-(4-NITRO-PHENOXY)-2H-PYRIDAZIN-3-ONE AND 6-(4-AMINO-PHENOXY)-2H-PYRIDAZIN-3-ONE DERIVATIVES AS INTERMEDIATES OF THYROID HORMONE ANALOGUES**
VERFAHREN ZUR HERSTELLUNG VON 6-(4-NITRO-PHENOXY)-2H-PYRIDAZIN-3-ON- UND 6-(4-AMINO-PHENOXY)-2H-PYRIDAZIN-3-ON-DERIVATE ALS ZWISCHENPRODUKTE VON THYROID-HORMON-ANALOGEN
PROCÉDÉ POUR LA PRÉPARATION DE DÉRIVÉS DE 6-(4-NITRO-PHÉNOXY)-2H-PYRIDAZIN-3-ONE ET 6-(4-AMINO-PHÉNOXY)-2H-PYRIDAZIN-3-ONE EN TANT QU'INTERMEDIAIRES D'ANALOGUES DU HORMONE DE THYROID

(30) Priority: 23.10.2020 US 202063104898 P; 18.02.2021 US 202163150616 P
(43) Date of publication of application: 30.08.2023
(73) Proprietor: Madrigal Pharmaceuticals, Inc., West Conshohocken, PA 19428 (US)
(72) Inventor: CONFALONE, Pasquale N., Wilmington, Delaware 19807 (US); VELLEKOOP, A. Samuel, Altamont, New York 12009 (US)
(74) Representative: Carpmaels & Ransford LLP
(86) International application number: PCT/US2021/055865
(87) International publication number: WO 2022/087141

(56) References cited:
- WO-A1-2019/240938
- WO-A1-2020/073974
- WO-A1-2020/123827
- WO-A2-2006/050389
- NIKOLOVA IVANKA ET AL: "Anti-enteroviral activity of new MDL-860 analogues: Synthesis, in vitro/in vivo studies and QSAR analysis", BIOORGANIC CHEMISTRY, vol. 85, April 2019 (2019-04-01), US, pages 487 - 497, XP055878140, ISSN: 0045-2068, DOI: 10.1016/j.bioorg.2019.02.020
- IVANKA NIKOLOVA ET AL: "Supplementary Data: Anti-enteroviral activity of new MDL-860 analogues: Synthesis, in vitro/in vivo studies and QSAR analysis", BIOORGANIC CHEMISTRY, vol. 85, April 2019 (2019-04-01), US, pages S1 - S165, XP009532653, ISSN: 0045-2068, DOI: 10.1016/j.bioorg.2019.02.020

## Description

### RELATED APPLICATIONS

This application claims priority to, and the benefit of, U.S. Application Nos. 63/104,898, filed October 23, 2020, and 63/150,616, filed on February 18, 2021.

### FIELD OF INVENTION

The invention relates to methods for preparing pyridazinone derivatives.

### BACKGROUND

Thyroid hormones are critical for normal growth and development and for maintaining metabolic homeostasis. Circulating levels of thyroid hormones are tightly regulated by feedback mechanisms in the hypothalamus/pituitary/thyroid (HPT) axis. Thyroid dysfunction leading to hypothyroidism or hyperthyroidism clearly demonstrates that thyroid hormones exert profound effects on cardiac function, body weight, metabolism, metabolic rate, body temperature, cholesterol, bone, muscle, and behavior.

The development of thyroid hormone analogs which avoid the undesirable effects of hyperthyroidism and hypothyroidism while maintaining the beneficial effects of thyroid hormones would open new avenues of treatment for patients with metabolic disease such as obesity, hyperlipidemia, hypercholesterolemia, diabetes and other disorders and diseases such as liver steatosis and Nonalcoholic steatohepatitis (NASH), atherosclerosis, cardiovascular diseases, hypothyroidism, thyroid cancer, thyroid diseases, resistance to thyroid hormone and related disorders and diseases.

There exists an unmet need to develop new methods of preparing pyridazinone compounds as thyroid hormone analogs.
Nikolova Ivanka et. al., Bioorganic Chemistry, vol. 85, April 2019, p.487-497, XP55878140, US, ISSN: 0045-2068 describes analogues of the anti-viral agent MDL-860 (a 5-nitro-2-phenoxybenzonitrile). WO 2020/123827 describes compounds that can serve as agonists of THRP receptors, and preparation methods and uses thereof. WO 2020/073974 describes thyroid hormone receptor (TR) agonists, methods of preparing the same and method of using the same for treating diseases or disorders modulated by TR agonists. WO 2006/050389 describes pyridazine compounds and related heterocyclic derivatives, compositions comprising the
same, and methods of making and using the same, for modulation of cellular pathways, for treatment or prevention of inflammatory diseases, and for research, drug screening, and therapeutic applications.

### SUMMARY

The present invention, in part, provides methods for synthesizing thyroid hormone analogs such as pyridazinone compounds and salts thereof. For example, the present invention provides non-Grignard methods for synthesizing thyroid hormone analogs such as pyridazinone compounds and salts thereof. The present invention also relates to intermediates for synthesizing pyridazinone compounds or salts thereof.

Invention provides a method of synthesizing a compound of formula V or Va:

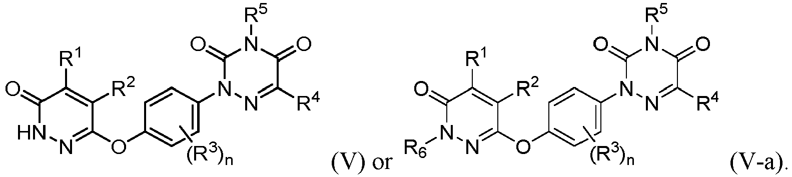

In one aspect, the present invention provides a method comprising contacting a compound of formula (I), a tautomer or a salt thereof: 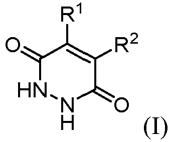 with a compound of formula (II) or a salt thereof: 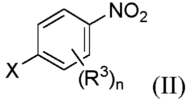 in a first organic solvent in the presence of a base, to form a compound of formula (III) or a salt thereof: 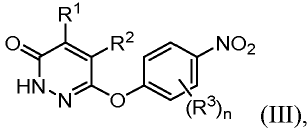 and
(A) contacting the compound of formula (III) or a salt thereof with a second organic solvent and a reducing agent to form a compound of formula (IV) or a salt thereof: 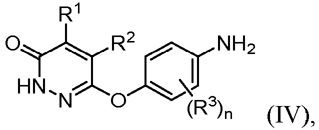 wherein:
   R¹ and R² are each independently hydrogen, deuterium, halogen, -CN, -OH, -OR^{a}, - SH, -SR^{a}, -S(=O)R^{a}, -S(=O)₂R^{a}, -NO₂, -NR^{b}R^{c}, -NHS(=O)₂R^{a}, -S(=O)₂NR^{b}R^{c}, -C(=O)R^{a}, - OC(=O)R^{a}, -C(=O)OR^{b}, -OC(=O)OR^{b}, -C(=O)NR^{b}R^{c}, -OC(=O)NR^{b}R^{c}, -NR^{b}C(=O)NR^{b}R^{c}, - NR^{b}C(=O)R^{a}, -NR^{b}C(=O)OR^{b}, C₁-C₆ alkyl, C₁-C₆ deuteroalkyl, C₁-C₆ haloalkyl, C₄-C₆ hydroxyalkyl, C₁-C₆ aminoalkyl, C₂-C₆ alkenyl, C₂-C₆ alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OR^{a}, -NR^{b}R^{c}, -C(=O)R^{a}, -C(=O)OR^{b}, -C(=O)NR^{b}R^{c}, C₁-C₆ alkyl, or C₁-C₆ haloalkyl;
   each R³ is independently hydrogen, deuterium, halogen, -CN, -OH, -OR^{a}, -SH, -SR^{a}, - S(=O)R^{a}, -S(=O)₂R^{a}, -NO₂, -NR^{b}R^{c}, -NHS(=O)₂R^{a}, -S(=O)₂NR^{b}R^{c}, -C(=O)R^{a}, -OC(=O)R^{a}, - C(=O)OR^{b}, -OC(=O)OR^{b}, -C(=O)NR^{b}R^{c}, -OC(=O)NR^{b}R^{c}, -NR^{b}C(=O)NR^{b}R^{c}, -NR^{b}C(=O)R^{a}, -NR^{b}C(=O)OR^{b}, C₁-C₆ alkyl, C₁-C₆ deuteroalkyl, C₁-C₆ haloalkyl, C₁-C₆ hydroxyalkyl, C₁-C₆ aminoalkyl, C₂-C₆ alkenyl, C₂-C₆ alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OR^{a}, -NR^{b}R^{c}, -C(=O)R^{a}, -C(=O)OR^{b}, -C(=O)NR^{b}R^{c}, C₁-C₆ alkyl, or C₁-C₆ haloalkyl;
   each R^{a} is independently C₁-C₆ alkyl, C₁-C₆ deuteroalkyl, C₁-C₆ haloalkyl, C₁-C₆ hydroxyalkyl, C₁-C₆ aminoalkyl, C₂-C₆ alkenyl, C₂-C₆ alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OCH₃, -NH₂, -C(=O)CH₃, -C(=O)OH, -C(=O)OCH₃, C₁-C₆alkyl, or C₁-C₆ haloalkyl;
   each R^{b} is independently hydrogen, deuterium, C₁-C₆ alkyl, C₁-C₆ deuteroalkyl, C₁-C₆ haloalkyl, C₁-C₆ hydroxyalkyl, C₁-C₆ aminoalkyl, C₂-C₆ alkenyl, C₂-C₆ alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OCH₃, -NH₂, -C(=O)CH₃, -C(=O)OH, - C(=O)OCH₃, C₁-C₆ alkyl, or C₁-C₆ haloalkyl;
   each R^{c} is independently hydrogen, deuterium, C₁-C₆ alkyl, C₁-C₆ deuteroalkyl, C₁-C₆ haloalkyl, C₁-C₆ hydroxyalkyl, C₁-C₆ aminoalkyl, C₂-C₆ alkenyl, C₂-C₆ alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OCH₃, -NH₂, -C(=O)CH₃, -C(=O)OH, - C(=O)OCH₃, C₁-C₆ alkyl, or C₁-C₆ haloalkyl;
   X is halogen; and
   n is 0, 1, 2, 3, or 4, or
(B) contacting the compound of formula (III) or a salt thereof with R⁶X to form a compound of formula (III-a) or a salt thereof: 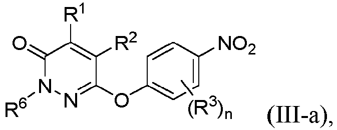 wherein:
   R⁶ is -CN, -OH, -OR^{a}, -S(=O)R^{a}, -S(=O)₂R^{a}, -S(=O)₂NR^{b}R^{c}, -C(=O)R^{a}, -OC(=O)R^{a}, - C(=O)OR^{b}, -C(=O)NR^{b}R^{c}, C₁-C₆ alkyl, C₁-C₆ deuteroalkyl, C₁-C₆ haloalkyl, C₁-C₆ hydroxyalkyl, C₁-C₆ aminoalkyl, C₂-C₆ alkenyl, C₂-C₆ alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OR^{a}, -NR^{b}R^{c}, -C(=O)R^{a}, -C(=O)OR^{b}, -C(=O)NR^{b}R^{c}, C₁-C₆ alkyl, or C₁-C₆ haloalkyl, and
   contacting the compound of formula (III-a) or a salt thereof with a second organic solvent and a reducing agent to form a compound of formula (IV-a) or a salt thereof:

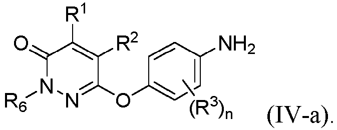

In some embodiments, the first organic solvent comprises dimethylformamide (DMF), dimethylacetamide (DMAC), dimethyl sulfoxide (DMSO), acetonitrile, tetrahydrofuran (THF), dichloromethane (DCM), dioxane, or acetone.

In some embodiments, the base comprises sodium carbonate (Na₂CO₃), sodium bicarbonate (NaHCO₃), potassium carbonate (K₂CO₃), or potassium bicarbonate (KHCO₃).

In some embodiments, the contacting occurs at room temperature or above room temperature.

In some embodiments, the reducing agent comprises hydrogen (H₂) gas and palladium on carbon (Pd/C), H₂ gas and Raney^{®} nickel, H₂ gas and platinum (IV) oxide, ferrous chloride, or stannous chloride.

In some embodiments, the contacting occurs at room temperature or above room temperature.

In one aspect, the present disclosure provides a method comprising contacting the compound of formula (IV) or a salt thereof with R⁴CH₂C(O)N(R⁵)C(O)OCH₂CH₃ to form a compound of formula (V) or a salt thereof: 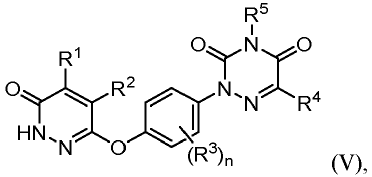 wherein:
R⁴ is hydrogen, deuterium, halogen, -CN, -OH, -OR^{a}, -SH, -SR^{a}, -S(=O)R^{a}, -S(=O)₂R^{a}, -NO₂, -NR^{b}R^{c}, -NHS(=O)₂R^{a}, -S(=O)₂NR^{b}R^{c}, -C(=O)R^{a}, -OC(=O)R^{a}, -C(=O)OR^{b}, -OC(=O)OR^{b}, - C(=O)NR^{b}R^{c}, -OC(=O)NR^{b}R^{c}, -NR^{b}C(=O)NR^{b}R^{c}, -NR^{b}C(=O)R^{a}, -NR^{b}C(=O)OR^{b}, C₁-C₆ alkyl, C₁-C₆ deuteroalkyl, C₁-C₆ haloalkyl, C₁-C₆ hydroxyalkyl, C₁-C₆ aminoalkyl, C₂-C₆ alkenyl, C₂-C₆ alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OR^{a}, -NR^{b}R^{c}, -C(=O)R^{a}, -C(=O)OR^{b}, -C(=O)NR^{b}R^{c}, C₁-C₆ alkyl, or C₁-C₆ haloalkyl; and
R⁵ is hydrogen, deuterium, halogen, -CN, -OH, -OR^{a}, -S(=O)R^{a}, -S(=O)₂R^{a}, -S(=O)₂NR^{b}R^{c}, - C(=O)R^{a}, -OC(=O)R^{a}, -C(=O)OR^{b}, -C(=O)NR^{b}R^{c}, C₁-C₆ alkyl, C₁-C₆ deuteroalkyl, C₁-C₆ haloalkyl, C₁-C₆ hydroxyalkyl, C₁-C₆ aminoalkyl, C₂-C₆ alkenyl, C₂-C₆ alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OR^{a}, -NR^{b}R^{c}, -C(=O)R^{a}, - C(=O)OR^{b}, -C(=O)NR^{b}R^{c}, C₁-C₆ alkyl, or C₁-C₆ haloalkyl.

In some embodiments, the method comprises contacting a compound of formula (IV) or a salt thereof with R⁴CH₂C(O)N(R⁵)C(O)OCH₂CH₃ in the presence of an oxidizing agent and an acid to form a compound of formula (IV-int) or a salt thereof:

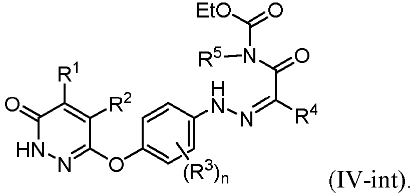

In some embodiments, the oxidizing agent is sodium nitrite (NaNO₂) or potassium nitrite (KNO₃). In some embodiments, the acid is hydrochloric acid (HCl) or acetic acid (AcOH).

In some embodiments, the method comprises contacting the compound of formula (IV-int) with a base to form a compound of formula (V). In some embodiments, the base is sodium acetate (NaOAc) or potassium acetate (KOAc).

In some embodiments, the reducing agent comprises H₂ gas and Pd/C, H₂ gas and Raney^{®} nickel, H₂ gas and platinum (IV) oxide, ferrous chloride, or stannous chloride.

In some embodiments, the contacting occurs at room temperature or above room temperature.

In one aspect, the present disclosure provides a method comprising contacting the compound of formula (IV-a) or a salt thereof with R⁴CH₂C(O)N(R⁵)C(O)OCH₂CH₃ to form a compound of formula (V-a) or a salt thereof: 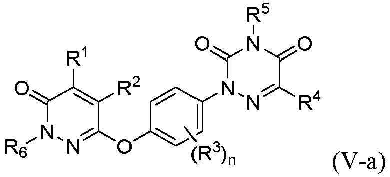 wherein:
R⁴ is hydrogen, deuterium, halogen, -CN, -OH, -OR^{a}, -SH, -SR^{a}, -S(=O)R^{a}, - S(=O)₂R^{a}, -NO₂, -NR^{b}R^{c}, -NHS(=O)₂R^{a}, -S(=O)₂NR^{b}R^{c}, -C(=O)R^{a}, -OC(=O)R^{a}, -C(=O)OR^{b}, -OC(=O)OR^{b}, -C(=O)NR^{b}R^{c}, -OC(=O)NR^{b}R^{c}, -NR^{b}C(=O)NR^{b}R^{c}, -NR^{b}C(=O)R^{a}, - NR^{b}C(=O)OR^{b}, C₁-C₆ alkyl, C₁-C₆ deuteroalkyl, C₁-C₆ haloalkyl, C₁-C₆ hydroxyalkyl, C₁-C₆ aminoalkyl, C₂-C₆ alkenyl, C₂-C₆ alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, - OR^{a}, -NR^{b}R^{c}, -C(=O)R^{a}, -C(=O)OR^{b}, -C(=O)NR^{b}R^{c}, C₁-C₆ alkyl, or C₁-C₆ haloalkyl; and
R⁵ is hydrogen, deuterium, halogen, -CN, -OH, -OR^{a}, -S(=O)R^{a}, -S(=O)₂R^{a}, - S(=O)₂NR^{b}R^{c}, -C(=O)R^{a}, -OC(=O)R^{a}, -C(=O)OR^{b}, -C(=O)NR^{b}R^{c}, C₁-C₆ alkyl, C₁-C₆ deuteroalkyl, C₁-C₆ haloalkyl, C₁-C₆ hydroxyalkyl, C₁-C₆ aminoalkyl, C₂-C₆ alkenyl, C₂-C₆ alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OR^{a}, -NR^{b}R^{c}, -C(=O)R^{a}, - C(=O)OR^{b}, -C(=O)NR^{b}R^{c}, C₁-C₆ alkyl, or C₁-C₆ haloalkyl.

In some embodiments, the method comprises contacting a compound of formula (IV-a) or a salt thereof with R⁴CH₂C(O)N(R⁵)C(O)OCH₂CH₃ in the presence of an oxidizing agent and an acid to form a compound of formula (IV-a-int) or a salt thereof:

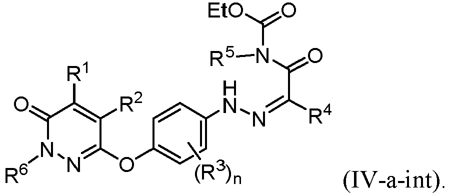

In some embodiments, the oxidizing agent is NaNO₂ or KNO₂. In some embodiments, the acid is HCl or AcOH.

In some embodiments, the method comprises contacting the compound of formula (IV-a-int) with a base to form a compound of formula (V-a). In some embodiments, the base is NaOAc or KOAc.

In some embodiments, the compound of formula (I) is 4-isopropylpyridazine-3,6-diol (compound 1-1), 4,5-dimethyl-1,2-dihydropyridazine-3,6-dione (compound 2-1), 4-methyl-1,2-dihydropyridazine-3,6-dione (compound 3-1), 4-phenyl-1,2-dihydropyridazine-3,6-dione (compound 4-1), or 4-(trifluoromethyl)-1,2-dihydropyridazine-3,6-dione (compound 5-1):

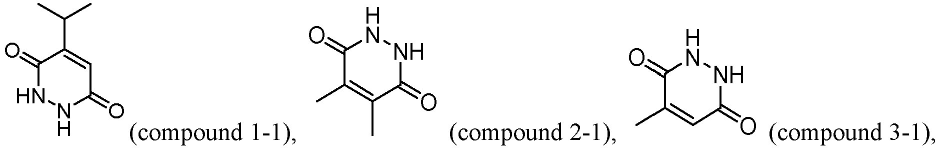

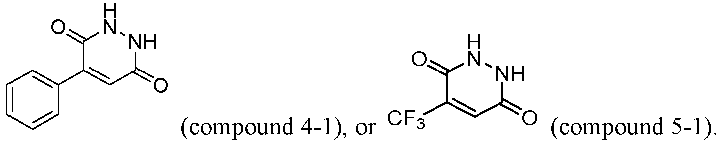

In some embodiments, the compound of formula (II) is 1,3-dichloro-2-fluoro-5-nitrobenzene (compound 1-2):

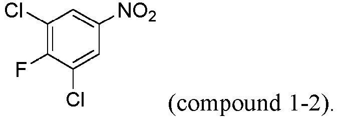

In some embodiments, the compound of formula (III) is 6-(2,6-dichloro-4-nitrophenoxy)-4-isopropylpyridazin-3(2H)-one (compound 1-4), 6-(2,6-dichloro-4-nitrophenoxy)-4,5-dimethylpyridazin-3(2H)-one (compound 2-2), 6-(2,6-dichloro-4-nitrophenoxy)-4-methylpyridazin-3(2H)-one (compound 3-2), 6-(2,6-dichloro-4-nitrophenoxy)-5-methylpyridazin-3(2H)-one (compound 3-3), 6-(2,6-dichloro-4-nitrophenoxy)-4-phenylpyridazin-3(2H)-one (compound 4-2), or 6-(2,6-dichloro-4-nitrophenoxy)-4-(trifluoromethyl)pyridazin-3(2H)-one (compound 5-2):

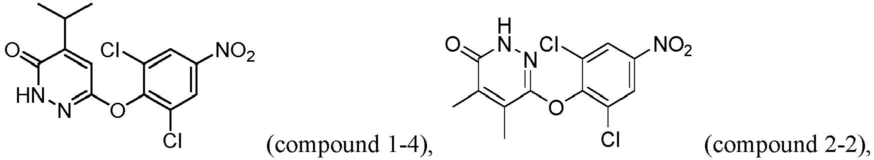

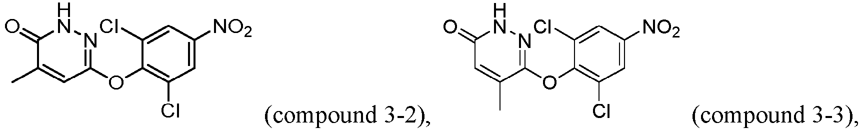

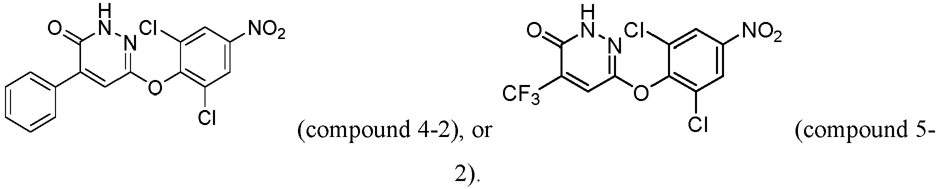

In some embodiments, the compound of formula (IV) is 6-(4-amino-2,6-dichlorophenoxy)-4-isopropylpyridazin-3(2H)-one (Int. 7), 6-(4-amino-2,6-dichlorophenoxy)-4,5-dimethylpyridazin-3(2H)-one (compound 2-3), 6-(4-amino-2,6-dichlorophenoxy)-4-methylpyridazin-3(2H)-one (compound 3-4), 6-(4-amino-2,6-dichlorophenoxy)-5-methylpyridazin-3(2H)-one (compound 3-5), 6-(4-amino-2,6-dichlorophenoxy)-4-phenylpyridazin-3(2H)-one (compound 4-3), 6-(4-amino-2,6-dichlorophenoxy)-4-(trifluoromethyl)pyridazin-3(2H)-one (compound 5-3), or 6-(4-amino-2,6-dichlorophenoxy)-5-isopropylpyridazin-3(2H)-one (compound 8-1): 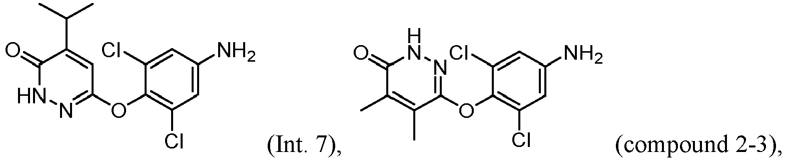 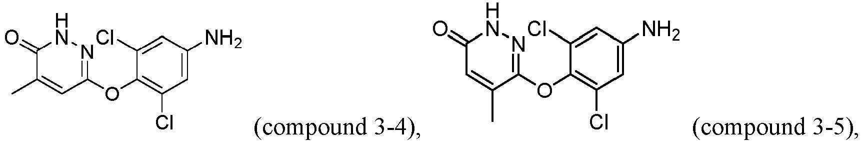 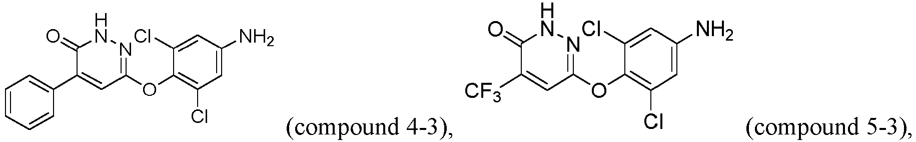 or

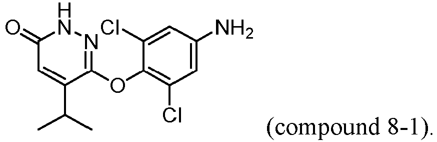

In some embodiments, the compound of formula (V) is 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile (MGL-3196).

In one aspect, the present disclosure provides a compound having the structure of

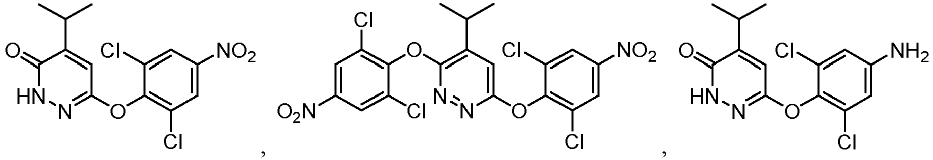

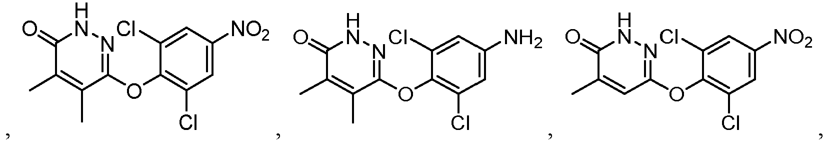

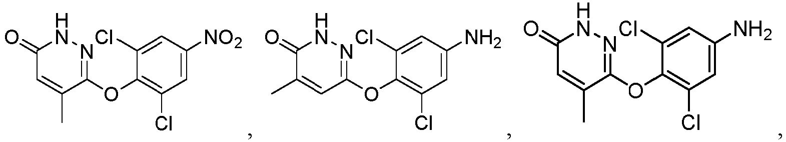

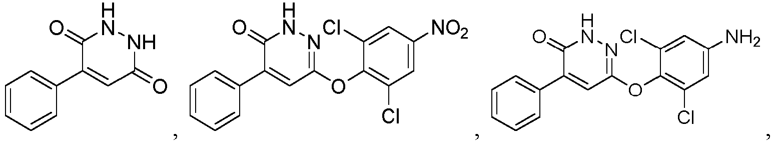

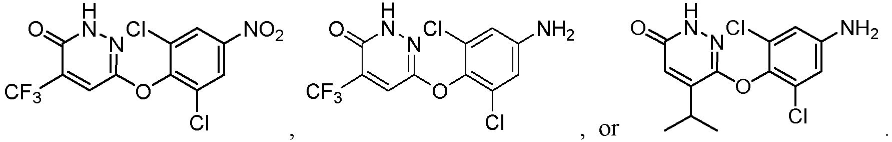

### DETAILED DESCRIPTION

The present disclosure provides non-Grignard methods for preparing pyridazinone derivatives. As compared to Grignard methods for preparing pyridazinone derivatives, the non-Grignard methods described herein are advantageous at least because they avoid the requirements to use hazardous, expensive Grignard reagents to manufacture the intermediates for the production of pyridazinone derivatives such as MGL-3196.

Some aspects of the present disclosure relate to one or more steps in a synthesis scheme according to Scheme 1, Scheme 2, or Scheme 3 below:

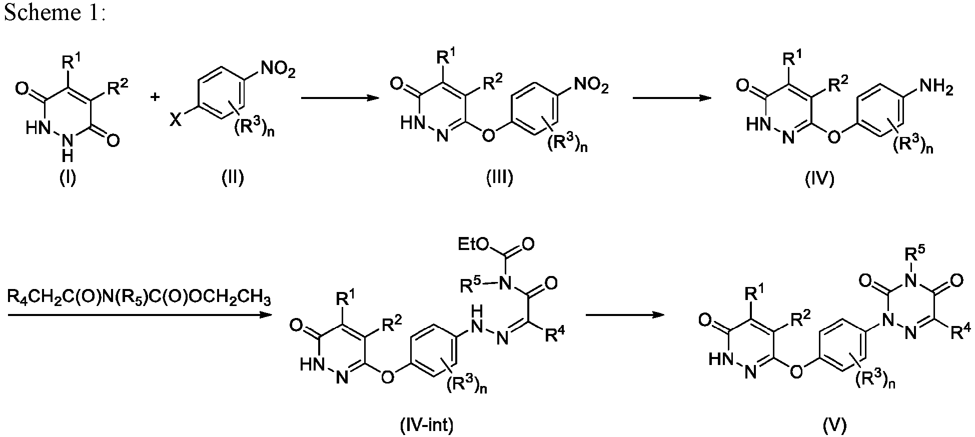

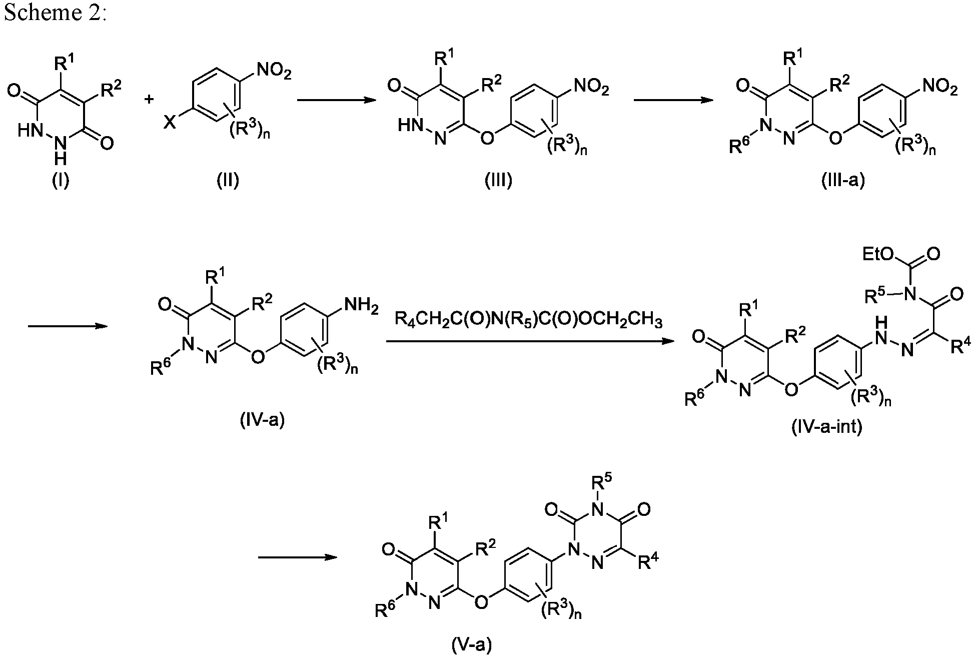

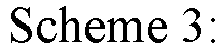

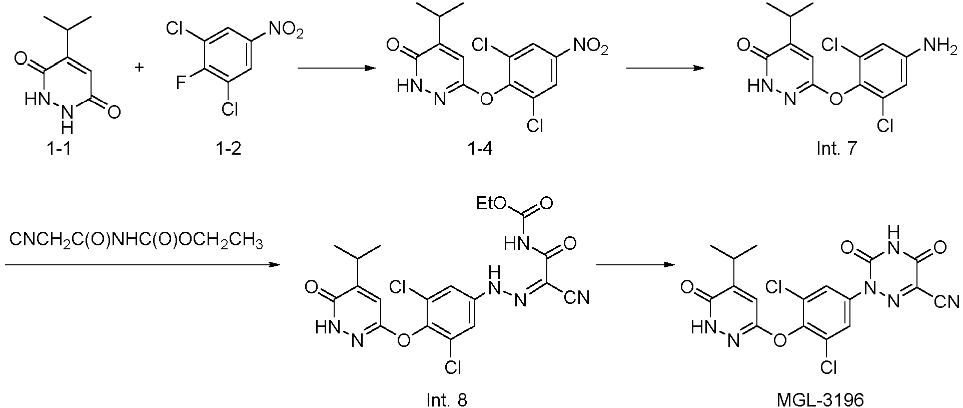

In one aspect, the present invention provides a method comprising contacting a compound of formula (I), a tautomer or a salt thereof: 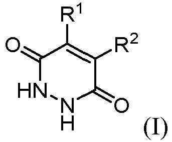 with a compound of formula (II) or a salt thereof: 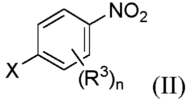 in a first organic solvent in the presence of a base, to form a compound of formula (III) or a salt thereof: 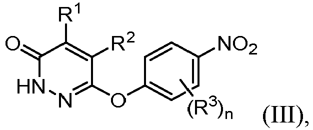 and
(A) contacting the compound of formula (III) or a salt thereof with a second organic solvent and a reducing agent to form a compound of formula (IV) or a salt thereof: 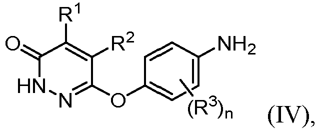 wherein:
   R¹ and R² are each independently hydrogen, deuterium, halogen, -CN, -OH, -OR^{a}, - SH, -SR^{a}, -S(=O)R^{a}, -S(=O)₂R^{a}, -NO₂, -NR^{b}R^{c}, -NHS(=O)₂R^{a}, -S(=O)₂NR^{b}R^{c}, -C(=O)R^{a}, -OC(=O)R^{a}, -C(=O)OR^{b}, -OC(=O)OR^{b}, -C(=O)NR^{b}R^{c}, -OC(=O)NR^{b}R^{c}, -NR^{b}C(=O)NR^{b}R^{c}, -NR^{b}C(=O)R^{a}, -NR^{b}C(=O)OR^{b}, C₁-C₆ alkyl, C₁-C₆ deuteroalkyl, C₁-C₆ haloalkyl, C₄-C₆ hydroxyalkyl, C₁-C₆ aminoalkyl, C₂-C₆ alkenyl, C₂-C₆ alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OR^{a}, -NR^{b}R^{c}, -C(=O)R^{a}, -C(=O)OR^{b}, -C(=O)NR^{b}R^{c}, C₁-C₆ alkyl, or C₁-C₆ haloalkyl;
   each R³ is independently hydrogen, deuterium, halogen, -CN, -OH, -OR^{a}, -SH, -SR^{a}, - S(=O)R^{a}, -S(=O)₂R^{a}, -NO₂, -NR^{b}R^{c}, -NHS(=O)₂R^{a}, -S(=O)₂NR^{b}R^{c}, -C(=O)R^{a}, -OC(=O)R^{a}, - C(=O)OR^{b}, -OC(=O)OR^{b}, -C(=O)NR^{b}R^{c}, -OC(=O)NR^{b}R^{c}, -NR^{b}C(=O)NR^{b}R^{c}, -NR^{b}C(=O)R^{a}, -NR^{b}C(=O)OR^{b}, C₁-C₆ alkyl, C₁-C₆ deuteroalkyl, C₁-C₆ haloalkyl, C₁-C₆ hydroxyalkyl, C₁-C₆ aminoalkyl, C₂-C₆ alkenyl, C₂-C₆ alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OR^{a}, -NR^{b}R^{c}, -C(=O)R^{a}, -C(=O)OR^{b}, -C(=O)NR^{b}R^{c}, C₁-C₆ alkyl, or C₁-C₆ haloalkyl;
   each R^{a} is independently C₁-C₆ alkyl, C₁-C₆ deuteroalkyl, C₁-C₆ haloalkyl, C₁-C₆ hydroxyalkyl, C₁-C₆ aminoalkyl, C₂-C₆ alkenyl, C₂-C₆ alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OCH₃, -NH₂, -C(=O)CH₃, -C(=O)OH, -C(=O)OCH₃, C₁-C₆alkyl, or C₁-C₆ haloalkyl;
   each R^{b} is independently hydrogen, deuterium, C₁-C₆ alkyl, C₁-C₆ deuteroalkyl, C₁-C₆ haloalkyl, C₁-C₆ hydroxyalkyl, C₁-C₆ aminoalkyl, C₂-C₆ alkenyl, C₂-C₆ alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OCH₃, -NH₂, -C(=O)CH₃, -C(=O)OH, - C(=O)OCH₃, C₁-C₆ alkyl, or C₁-C₆ haloalkyl;
   each R^{c} is independently hydrogen, deuterium, C₁-C₆ alkyl, C₁-C₆ deuteroalkyl, C₁-C₆ haloalkyl, C₁-C₆ hydroxyalkyl, C₁-C₆ aminoalkyl, C₂-C₆ alkenyl, C₂-C₆ alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OCH₃, -NH₂, -C(=O)CH₃, -C(=O)OH, - C(=O)OCH₃, C₁-C₆ alkyl, or C₁-C₆ haloalkyl;
   X is halogen; and
   n is 0, 1, 2, 3, or 4, or
(B) contacting the compound of formula (III) or a salt thereof with R⁶X to form a compound of formula (III-a) or a salt thereof: 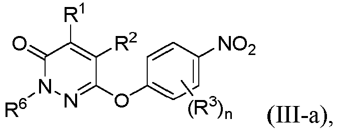 wherein:
   R⁶ is -CN, -OH, -OR^{a}, -S(=O)R^{a}, -S(=O)₂R^{a}, -S(=O)₂NR^{b}R^{c}, -C(=O)R^{a}, -OC(=O)R^{a}, - C(=O)OR^{b}, -C(=O)NR^{b}R^{c}, C₁-C₆ alkyl, C₁-C₆ deuteroalkyl, C₁-C₆ haloalkyl, C₁-C₆ hydroxyalkyl, C₁-C₆ aminoalkyl, C₂-C₆ alkenyl, C₂-C₆ alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OR^{a}, -NR^{b}R^{c}, -C(=O)R^{a}, -C(=O)OR^{b}, -C(=O)NR^{b}R^{c}, C₁-C₆ alkyl, or C₁-C₆ haloalkyl, and
   contacting the compound of formula (III-a) or a salt thereof with a second organic solvent and a reducing agent to form a compound of formula (IV-a) or a salt thereof:

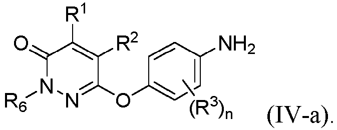

In some embodiments, n is 0. In some embodiments, n is 1. In some embodiments, n is 2. In some embodiments, n is 3. In some embodiments, n is 4.

In some embodiments, each alkyl, alkenyl, alkynyl, cycloalkyl, heterocycloalkyl, aryl, and heteroaryl in R¹ is independently optionally substituted with one, two, or three oxo, deuterium, halogen, -CN, -OH, -OR^{a}, -NR^{b}R^{c}, -C(=O)R^{a}, -C(=O)OR^{b}, -C(=O)NR^{b}R^{c}, C₁-C₆ alkyl, or C₁-C₆ haloalkyl.

In some embodiments, R¹ is hydrogen or deuterium. In some embodiments, R¹ is hydrogen.

In some embodiments, R¹ is halogen, -CN, -OH, -OR^{a}, - NR^{b}R^{c}, -C(=O)R^{a}, - C(=O)OR^{b}, -C(=O)NR^{b}R^{c}, C₄-C₆ alkyl, C₁-C₆ deuteroalkyl, C₁-C₆ haloalkyl, C₄-C₆ hydroxyalkyl, C₁-C₆ aminoalkyl, cycloalkyl, heterocycloalkyl, aryl, or heteroaryl; wherein each alkyl, cycloalkyl, heterocycloalkyl, aryl, and heteroaryl is independently optionally substituted with one or more oxo, deuterium, halogen, -CN, -OH, -OR^{a}, -NR^{b}R^{c}, C₁-C₆ alkyl, or C₁-C₆ haloalkyl.

In some embodiments, R¹ is C₄-C₆ alkyl, C₁-C₆ deuteroalkyl, C₁-C₆ haloalkyl, cycloalkyl, or heterocycloalkyl; wherein each alkyl, cycloalkyl, and heterocycloalkyl is independently optionally substituted with one or more oxo, deuterium, halogen, -CN, -OH, - OR^{a}, -NR^{b}R^{c}, C₁-C₆ alkyl, or C₁-C₆ haloalkyl.

In some embodiments, R¹ is C₄-C₆ alkyl, C₁-C₆ deuteroalkyl, C₁-C₆ haloalkyl, cycloalkyl, or heterocycloalkyl; wherein each alkyl, cycloalkyl, and heterocycloalkyl is independently optionally substituted with one or more halogen. In some embodiments, R¹ is C₁-C₆ deuteroalkyl.

In some embodiments, R¹ is C₁-C₆ alkyl, C₂-C₆ alkenyl, or C₂-C₆ alkynyl.

In some embodiments, R¹ is C₁-C₆ alkyl. In some embodiments, R¹ is methyl. In some embodiments, R¹ is ethyl. In some embodiments, R¹ is propyl. In some embodiments, R¹ is butyl. In some embodiments, R¹ is isopropyl. In some embodiments, R¹ is iso-butyl. In some embodiments, R¹ is sec-butyl. In some embodiments, R¹ is tert-butyl. In some embodiments, R¹ is pentyl. In some embodiments, R¹ is iso-pentyl. In some embodiments, R¹ is hexyl. In some embodiments, R¹ is iso-hexyl.

In some embodiments, R¹ is C₂-C₆ alkenyl. In some embodiments, R¹ is C₂ alkenyl. In some embodiments, R¹ is C₃ alkenyl. In some embodiments, R¹ is C₄ alkenyl. In some embodiments, R¹ is C₅ alkenyl. In some embodiments, R¹ is C₆ alkenyl.

In some embodiments, R¹ is C₂-C₆ alkynyl. In some embodiments, R¹ is C₂ alkynyl. In some embodiments, R¹ is C₃ alkynyl. In some embodiments, R¹ is C₄ alkynyl. In some embodiments, R¹ is C₅ alkynyl. In some embodiments, R¹ is C₆ alkynyl.

In some embodiments, each alkyl, alkenyl, alkynyl, cycloalkyl, heterocycloalkyl, aryl, and heteroaryl in R² is independently optionally substituted with one, two, or three oxo, deuterium, halogen, -CN, -OH, -OR^{a}, -NR^{b}R^{c}, -C(=O)R^{a}, -C(=O)OR^{b}, -C(=O)NR^{b}R^{c}, C₁-C₆ alkyl, or C₁-C₆ haloalkyl.

In some embodiments, R² is hydrogen, deuterium, C₁-C₆ alkyl, or C₁-C₆ haloalkyl. In some embodiments, R² is hydrogen. In some embodiments, R² is deuterium.

In some embodiments, each alkyl, alkenyl, alkynyl, cycloalkyl, heterocycloalkyl, aryl, and heteroaryl in R³ is independently optionally substituted with one, two, or three oxo, deuterium, halogen, -CN, -OH, -OR^{a}, -NR^{b}R^{c}, -C(=O)R^{a}, -C(=O)OR^{b}, -C(=O)NR^{b}R^{c}, C₁-C₆ alkyl, or C₁-C₆ haloalkyl.

In some embodiments, each R³ is independently hydrogen, halogen, C₁-C₆ alkyl, or C₁-C₆ haloalkyl. In some embodiments, each R³ is independently halogen, such as halogen, fluorine, chlorine, bromine, and iodine. In some embodiments, one of R³ is deuterium. In some embodiments, each R³ is independently hydrogen, deuterium, halogen, C₁-C₆ alkyl, or C₁-C₆ haloalkyl. In some embodiments, each R³ is independently deuterium, halogen, or C₁-C₆ alkyl. In some embodiments, each R³ is independently deuterium or halogen.

In some embodiments, X is halogen. In some embodiments, X is fluorine. In some embodiments, X is chlorine. In some embodiments, X is bromine. In some embodiments, X is iodine.

In some embodiments, the compound of formula (I) is a compound of formula (I-a): 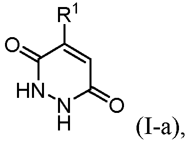 a tautomer or a salt thereof, wherein R¹ is as described herein for formula (I).

In some embodiments, the compound of formula (I) is a compound of formula (I-b): 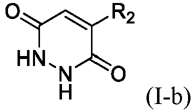 a tautomer, or a salt thereof, wherein R² is as described herein for formula (I).

In some embodiments, the compound of formula (II) is a compound of formula (II-a): 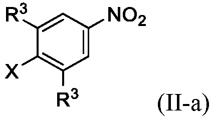 or a salt thereof, wherein X and R³ is as described herein for formula (II).

In some embodiments, the compound of formula (II) is a compound of formula (II-b): 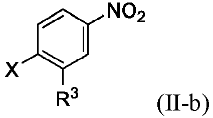 or a salt thereof, wherein X and R³ is as described herein for formula (II).

In some embodiments, the compound of formula (II) is a compound of formula (II-c): 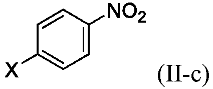 or a salt thereof, wherein X is as described herein for formula (II).

In some embodiments, the compound of formula (III) is a compound of formula (III-b): 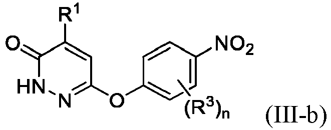 or a salt thereof, wherein R¹, R³, and n are as described herein for formula (III).

In some embodiments, the compound of formula (III) is a compound of formula (III-d): 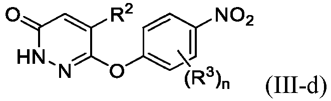 or a salt thereof, wherein R², R³, and n are as described herein for formula (III).

In some embodiments, the compound of formula (III) is a compound of formula (III-f): 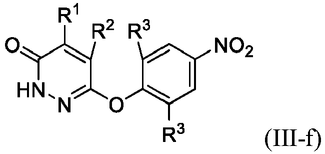 or a salt thereof, wherein R¹, R², and R³ are as described herein for formula (III).

In some embodiments, the compound of formula (III) is a compound of formula (III-h): 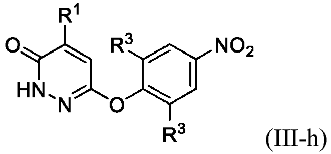 or a salt thereof, wherein R¹ and R³ are as described herein for formula (III).

In some embodiments, a compound of formula (I) is 4-isopropylpyridazine-3,6-diol (compound 1-1):

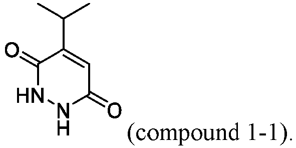

In some embodiments, a compound of formula (II) is 1,3-dichloro-2-fluoro-5-nitrobenzene (compound 1-2):

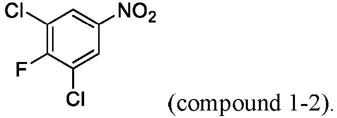

In some embodiments, a compound of formula (III) is 6-(2,6-dichloro-4-nitrophenoxy)-4-isopropylpyridazin-3(2H)-one (compound 1-4):

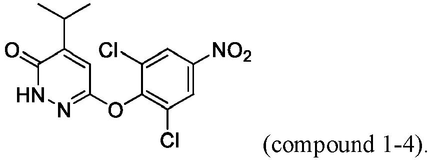

The first organic solvent used in the synthesis of the compound of formula (III) can comprise DMF, DMAC, DMSO, acetonitrile, THF, DCM, dioxane, acetone, or a combination thereof. In some embodiments, the first organic solvent used in the synthesis of the compound of formula (III) is DMF, DMAC, DMSO, acetonitrile, THF, DCM, dioxane, acetone, or a combination thereof. In some embodiments, the first organic solvent comprises DMF. In some embodiments, the first organic solvent is DMF.

The base used in the synthesis of the compound of formula (III) can comprise Na₂CO₃, NaHCO₃, K₂CO₃, KHCO₃, or a combination thereof. In some embodiments, the base used in the synthesis of the compound of formula (III) is Na₂CO₃, NaHCO₃, K₂CO₃, KHCO₃, or a combination thereof. In some embodiments, the base is added as a solid.

In some embodiments, the compound of formula (IV) is a compound of formula (IV-b): 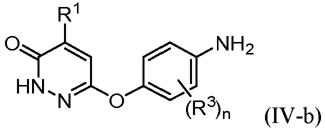 or a salt thereof, wherein R¹, R³, and n are as described herein for formula (IV).

In some embodiments, the compound of formula (IV) is a compound of formula (IV-d): 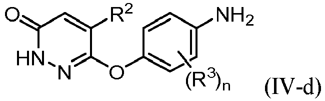 or a salt thereof, wherein R², R³, and n are as described herein for formula (IV).

In some embodiments, the compound of formula (IV) is a compound of formula (IV-f): 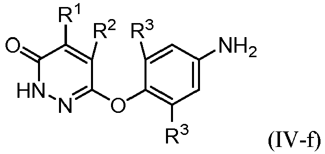 or a salt thereof, wherein R¹, R², and R³ are as described herein for formula (IV)

In some embodiments, the compound of formula (IV) is a compound of formula (IV-h): 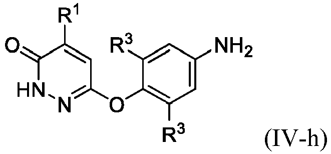 or a salt thereof, wherein R¹ and R³ are as described herein for formula (IV).

In some embodiments, the compound of formula (IV) is 6-(4-amino-2,6-dichlorophenoxy)-4-isopropylpyridazin-3(2H)-one (Int. 7):

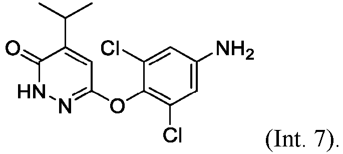

The second organic solvent used in the synthesis of the compound of formula (IV) can comprise DMF, DMAC, DMSO, acetonitrile, THF, DCM, dioxane, acetone, or a combination thereof. The second organic solvent used in the synthesis of the compound of formula (IV) is DMF, DMAC, DMSO, acetonitrile, THF, DCM, dioxane, acetone, or a combination thereof. In some embodiments, the second organic solvent comprises THF. In some embodiments, the second organic solvent is THF.

In some embodiments, the reducing agent used in the synthesis of the compound of formula (IV) can comprise H₂ gas and Pd/C, H₂ gas and Raney^{®} nickel, H₂ gas and platinum (IV) oxide, ferrous chloride, or stannous chloride. In some embodiments, the reducing agent used in the synthesis of the compound of formula (IV) is H₂ gas and Pd/C, H₂ gas and Raney^{®} nickel, H₂ gas and platinum (IV) oxide, ferrous chloride, or stannous chloride. In some embodiments, the reducing agent comprises H₂ gas and Pd/C. In some embodiments, the reducing agent is H₂ gas and Pd/C.

In some embodiments, the method further comprises contacting the compound of formula (IV) or a salt thereof with R⁴CH₂C(O)N(R⁵)C(O)OCH₂CH₃ to form a compound of formula (V) or a salt thereof: 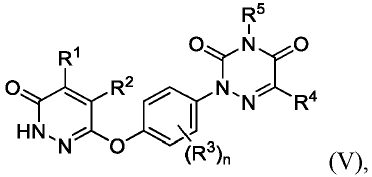 wherein:
R⁴ is hydrogen, deuterium, halogen, -CN, -OH, -OR^{a}, -SH, -SR^{a}, -S(=O)R^{a}, - S(=O)₂R^{a}, -NO₂, -NR^{b}R^{c}, -NHS(=O)₂R^{a}, -S(=O)₂NR^{b}R^{c}, -C(=O)R^{a}, -OC(=O)R^{a}, -C(=O)OR^{b}, -OC(=O)OR^{b}, -C(=O)NR^{b}R^{c}, -OC(=O)NR^{b}R^{c}, -NR^{b}C(=O)NR^{b}R^{c}, -NR^{b}C(=O)R^{a}, - NR^{b}C(=O)OR^{b}, C₁-C₆ alkyl, C₁-C₆ deuteroalkyl, C₁-C₆ haloalkyl, C₁-C₆ hydroxyalkyl, C₁-C₆ aminoalkyl, C₂-C₆ alkenyl, C₂-C₆ alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, - OR^{a}, -NR^{b}R^{c}, -C(=O)R^{a}, -C(=O)OR^{b}, -C(=O)NR^{b}R^{c}, C₁-C₆ alkyl, or C₁-C₆ haloalkyl; and
R⁵ is hydrogen, deuterium, halogen, -CN, -OH, -OR^{a}, -S(=O)R^{a}, -S(=O)₂R^{a}, - S(=O)₂NR^{b}R^{c}, -C(=O)R^{a}, -OC(=O)R^{a}, -C(=O)OR^{b}, -C(=O)NR^{b}R^{c}, C₁-C₆ alkyl, C₁-C₆ deuteroalkyl, C₁-C₆ haloalkyl, C₁-C₆ hydroxyalkyl, C₁-C₆ aminoalkyl, C₂-C₆ alkenyl, C₂-C₆ alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OR^{a}, -NR^{b}R^{c}, -C(=O)R^{a}, - C(=O)OR^{b}, -C(=O)NR^{b}R^{c}, C₁-C₆ alkyl, or C₁-C₆ haloalkyl.

In some embodiments, each alkyl, alkenyl, alkynyl, cycloalkyl, heterocycloalkyl, aryl, and heteroaryl in R⁴ is independently optionally substituted with one, two, or three oxo, deuterium, halogen, -CN, -OH, -OR^{a}, -NR^{b}R^{c}, -C(=O)R^{a}, -C(=O)OR^{b}, -C(=O)NR^{b}R^{c}, C₁-C₆ alkyl, or C₁-C₆ haloalkyl.

In some embodiments, R⁴ is hydrogen, deuterium or halogen.

In some embodiments, R⁴ is -CN.

In some embodiments, each alkyl, alkenyl, alkynyl, cycloalkyl, heterocycloalkyl, aryl, and heteroaryl in R⁵ is independently optionally substituted with one or more oxo, deuterium, halogen, -CN, -OH, -OR^{a}, -NR^{b}R^{c}, -C(=O)R^{a}, -C(=O)OR^{b}, -C(=O)NR^{b}R^{c}, C₁-C₆ alkyl, or C₁-C₆ haloalkyl.

In some embodiments, R⁵ is hydrogen or C₁-C₆ alkyl.

In some embodiments, R⁵ is hydrogen.

In some embodiments, the compound of formula (V) is a compound of formula (V-b): 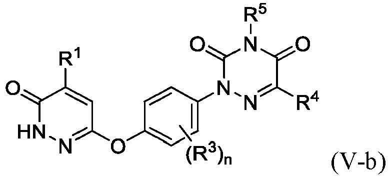 or a salt thereof, wherein R¹, R³, R⁴, R⁵, and n are as described herein for formula (V).

In some embodiments, the compound of formula (V) is a compound of formula (V-d): 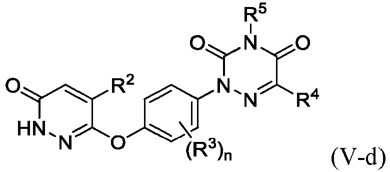 or a salt thereof, wherein R², R³, R⁴, R⁵, and n are as described herein for formula (V).

In some embodiments, the compound of formula (V) is a compound of formula (V-f): 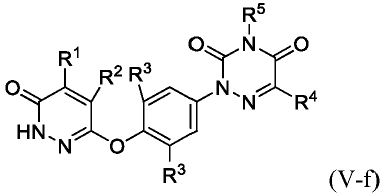 or a salt thereof, wherein R¹, R², R³, R⁴, and R⁵ are as described herein for formula (V).

In some embodiments, the compound of formula (V) is a compound of formula (V-h): 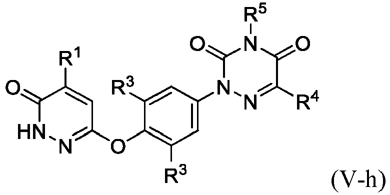 or a salt thereof, wherein R¹, R³, R⁴, and R⁵ are as described herein for formula (V).

In some embodiments, R⁴CH₂C(O)N(R⁵)C(O)OCH₂CH₃ is CNCH₂C(O)NHC(O)OCH₂CH₃.

In some embodiments, a compound of formula (V) is 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile (MGL-3196).

In some embodiments, prior to the formation of a compound of formula (V), the method further comprises contacting a compound of formula (IV) or a salt thereof with R⁴CH₂C(O)N(R⁵)C(O)OCH₂CH₃, as described above, in the presence of an oxidizing agent and an acid to form a compound of formula (IV-int) or a salt thereof:

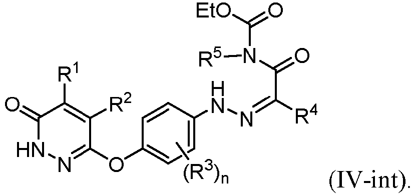

In some embodiments, the oxidizing agent used in the synthesis of the compound of formula (IV-int) can comprise NaNO₂, KNO₂, or a combination thereof. In some embodiments, the oxidizing agent used in the synthesis of the compound of formula (IV-int) is NaNO₂, KNO₂, or a combination thereof. In some embodiments, the acid used in the synthesis of the compound of formula (V-int) can comprise HCl, AcOH, or a combination thereof. In some embodiments, the acid used in the synthesis of the compound of formula (V-int) is HCl, AcOH, or a combination thereof.

In some embodiments, the method further comprises contacting the compound of formula (IV-int) with a base to form a compound of formula (V). In some embodiments, the base used in the synthesis of the compound of formula (V) can comprise sodium acetate (NaOAc), potassium acetate (KOAc), or a combination thereof. In some embodiments, the base used in the synthesis of the compound of formula (V) is NaOAc, KOAc, or a combination thereof.

In some embodiments, each alkyl, alkenyl, alkynyl, cycloalkyl, heterocycloalkyl, aryl, and heteroaryl in R⁶ is independently optionally substituted with one, two, or three oxo, deuterium, halogen, -CN, -OH, -OR^{a}, -NR^{b}R^{c}, -C(=O)R^{a}, -C(=O)OR^{b}, -C(=O)NR^{b}R^{c}, C₁-C₆ alkyl, or C₁-C₆ haloalkyl.

In some embodiments, R⁶ is C₁-C₆ alkyl.

In some embodiments, the compound of formula (III-a) is a compound of formula (III-c): 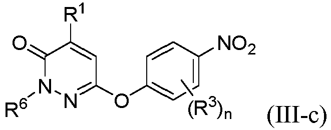 or a salt thereof, wherein R¹, R³, R⁶, and n are as described herein for formula (III-a).

In some embodiments, the compound of formula (III-a) is a compound of formula (III-e): 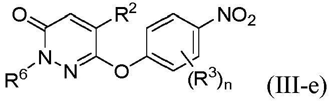 or a salt thereof, wherein R², R³, R⁶, and n are as described herein for formula (III-a).

In some embodiments, the compound of formula (III-a) is a compound of formula (III-g): 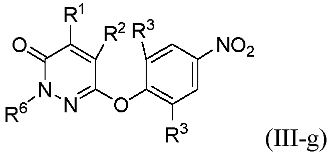 or a salt thereof, wherein R¹, R², R³, and R⁶ are as described herein for formula (III-a).

In some embodiments, the compound of formula (III-a) is a compound of formula (III-i): 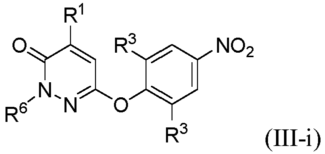 or a salt thereof, wherein R¹, R³, and R⁶ are as described herein for formula (III-a).

In some embodiments, the second organic solvent used in the synthesis of the compound of formula (IV-a) can comprise DMF, DMAC, DMSO, acetonitrile, THF, DCM, dioxane, acetone, or a combination thereof. In some embodiments, the second organic solvent used in the synthesis of the compound of formula (IV-a) is DMF, DMAC, DMSO, acetonitrile, THF, DCM, dioxane, acetone, or a combination thereof. In some embodiments, the second organic solvent comprises THF. In some embodiments, the second organic solvent is THF.

In some embodiments, the reducing agent used in the synthesis of the compound of formula (IV-a) can comprise H₂ gas and Pd/C, H₂ gas and Raney^{®} nickel, H₂ gas and platinum (IV) oxide, ferrous chloride, or stannous chloride. In some embodiments, the reducing agent used in the synthesis of the compound of formula (IV-a) is H₂ gas and Pd/C, H₂ gas and Raney^{®} nickel, H₂ gas and platinum (IV) oxide, ferrous chloride, or stannous chloride. In some embodiments, the reducing agent comprises H₂ gas and Pd/C. In some embodiments, the reducing agent is H₂ gas and Pd/C.

In some embodiments, the compound of formula (IV-a) is a compound of formula (IV-c): 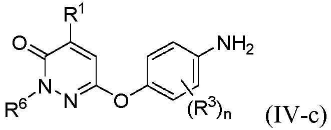 or a salt thereof, wherein R¹, R³, R⁶, and n are as described herein for formula (IV-a).

In some embodiments, the compound of formula (IV-a) is a compound of formula (IV-e): 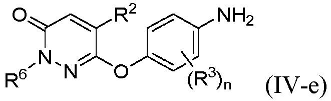 or a salt thereof, wherein R², R³, R⁶, and n are as described herein for formula (IV-a).

In some embodiments, the compound of formula (IV-a) is a compound of formula (IV-g): 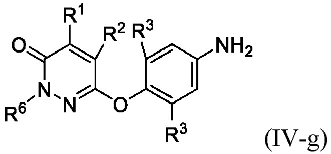 or a salt thereof, wherein R¹, R², R³, and R⁶ are as described herein for formula (IV-a).

In some embodiments, the compound of formula (IV-a) is a compound of formula (IV-i): 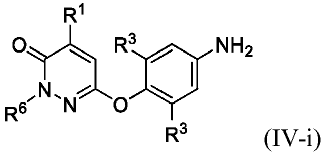 or a salt thereof, wherein R¹, R³, and R⁶ are as described herein for formula (IV-a).

In some embodiments, the method further comprises contacting the compound of formula (IV-a) or a salt thereof with R⁴CH₂C(O)N(R⁵)C(O)OCH₂CH₃, as described above, to form a compound of formula (V-a) or a salt thereof:

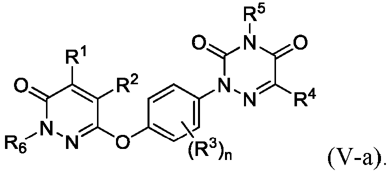

In some embodiments, prior to the formation of a compound of formula (V-a), the method comprises contacting a compound of formula (IV-a) or a salt thereof with R⁴CH₂C(O)N(R⁵)C(O)OCH₂CH₃, as described above, in the presence of an oxidizing agent and an acid to form a compound of formula (IV-a-int) or a salt thereof:

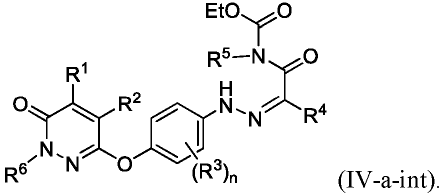

In some embodiments, the oxidizing agent used in the synthesis of the compound of formula (IV-a-int) can comprise NaNO₂, KNO₂, or a combination thereof. In some embodiments, the oxidizing agent used in the synthesis of the compound of formula (IV-a-int) is NaNO₂, KNO₂, or a combination thereof. In some embodiments, the acid used in the synthesis of the compound of formula (IV-a-int) can comprise HCl, AcOH, or a combination thereof. In some embodiments, the acid used in the synthesis of the compound of formula (IV-a-int) is HCl, AcOH, or a combination thereof.

In some embodiments, the method further comprises contacting the compound of formula (IV-a-int) with a base to form a compound of formula (V-a). In some embodiments, the base used in the synthesis of the compound of formula (V-a) can comprise NaOAc, KOAc, or a combination thereof. In some embodiments, the base used in the synthesis of the compound of formula (V-a) is NaOAc, KOAc, or a combination thereof.

In some embodiments, the compound of formula (V-a) is a compound of formula (V-c): 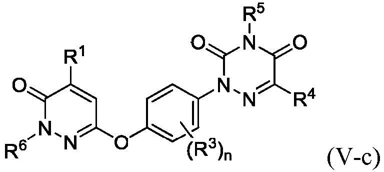 or a salt thereof, wherein R¹, R³, R⁴, R⁵, R⁶, and n are as described herein for formula (V-a).

In some embodiments, the compound of formula (V-a) is a compound of formula (V-e): 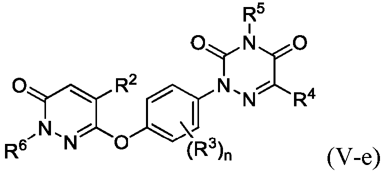 or a salt thereof, wherein R², R³, R⁴, R⁵, R⁶, and n are as described herein for formula (V-a).

In some embodiments, the compound of formula (V-a) is a compound of formula (V-g): 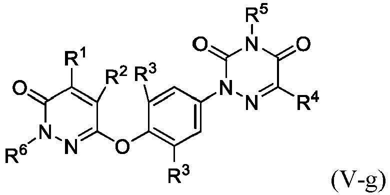 or a salt thereof, wherein R¹, R², R³, R⁴, R⁵, and R⁶ are as described herein for formula (V-a).

In some embodiments, the compound of formula (V-a) is a compound of formula (V-i): 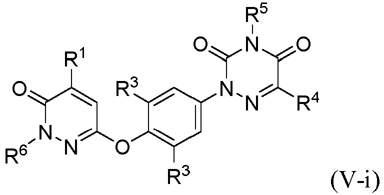 or a salt thereof, wherein R¹, R³, R⁴, R⁵, and R⁶ are as described herein for formula (V-a).

Embodiments of the compounds of formula (V) or (V-a) can be found in WO2019/240938.

In some embodiments, the compounds can be contacted at room temperature, above room temperature, or below room temperature. In some embodiments, the contacting occurs at room temperature.

In one aspect, the present disclosure provides a compound having the structure of 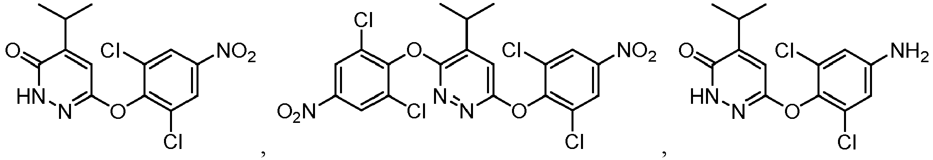 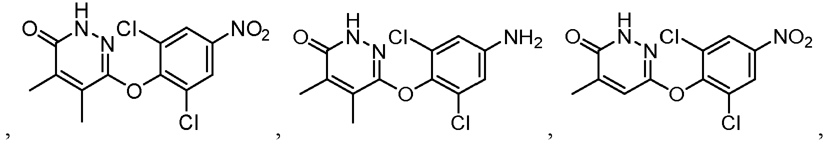 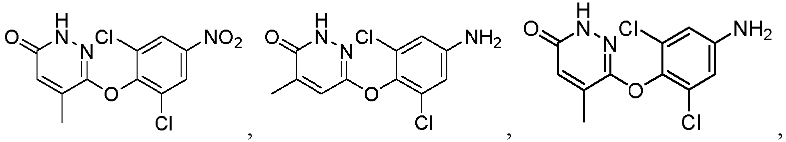 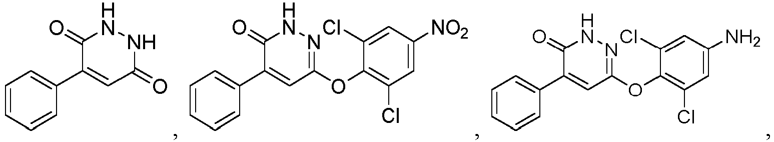 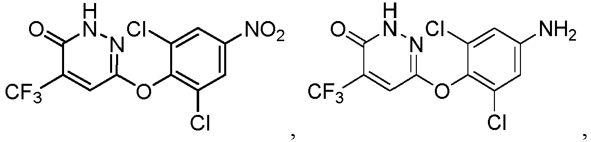 or

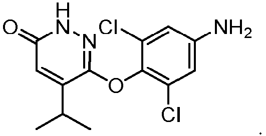

Examples of compounds of the present disclosure are shown in Table 1 below.

**Table 1. Compounds of the Present Disclosure**

| Compound Number | Compound Structure |
|---|---|
| 1-1 | 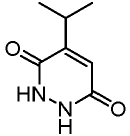 |
| 1-2 | 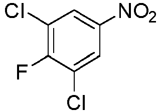 |
| 1-4 | 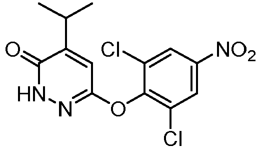 |
| 1-5 | 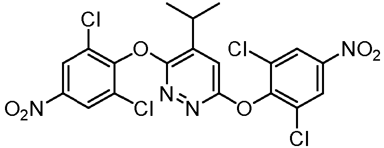 |
| Int. 7 | 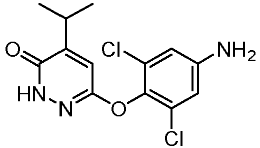 |
| Int. 8 | 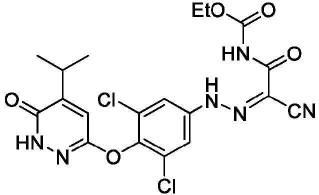 |
| MGL-3196 | 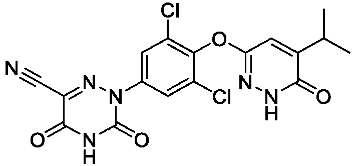 |
| 2-1 | 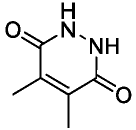 |
| 2-2 | 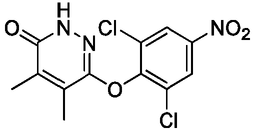 |
| 2-3 | 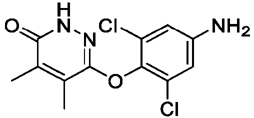 |
| 3-1 | 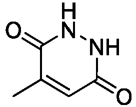 |
| 3-2 | 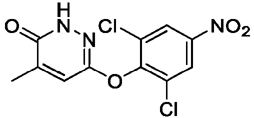 |
| 3-3 | 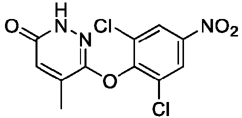 |
| 3-4 | 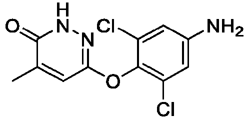 |
| 3-5 | 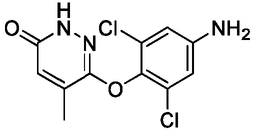 |
| 4-1 | 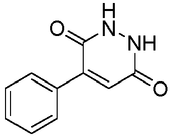 |
| 4-2 | 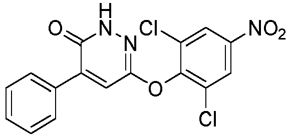 |
| 4-3 | 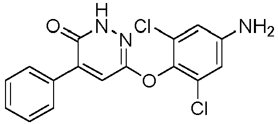 |
| 5-1 | 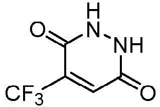 |
| 5-2 | 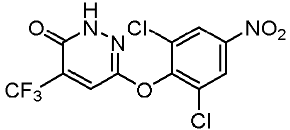 |
| 5-3 | 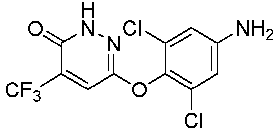 |
| 8-1 | 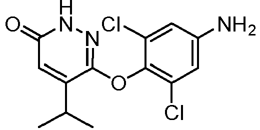 |

### Pharmaceutically Acceptable Salts

In some embodiments, the compounds described herein exist as their pharmaceutically acceptable salts. In some embodiments, such pharmaceutically acceptable salts are for use in methods of treating diseases. In some embodiments, such pharmaceutically acceptable salts are administered as pharmaceutical compositions for use in methods of treating diseases.

In some embodiments, the compounds described herein possess acidic or basic groups and therefor react with any of a number of inorganic or organic bases, and inorganic and organic acids, to form a pharmaceutically acceptable salt. In some embodiments, these salts are prepared *in situ* during the final isolation and purification of the compounds disclosed herein, or by separately reacting a purified compound in its free form with a suitable acid or base, and isolating the salt thus formed.

Examples of pharmaceutically acceptable salts include those salts prepared by reaction of the compounds described herein with a mineral, organic acid, or inorganic base, such salts including acetate, acrylate, adipate, alginate, aspartate, benzoate, benzenesulfonate, bisulfate, bisulfite, bromide, butyrate, butyn-l,4-dioate, camphorate, camphorsulfonate, caproate, caprylate, chlorobenzoate, chloride, citrate, cyclopentanepropionate, decanoate, digluconate, dihydrogenphosphate, dinitrobenzoate, dodecylsulfate, ethanesulfonate, formate, fumarate, glucoheptanoate, glycerophosphate, glycolate, hemisulfate, heptanoate, hexanoate, hexyne-l,6-dioate, hydroxybenzoate, g-hydroxybutyrate, hydrochloride, hydrobromide, hydroiodide, 2-hydroxyethanesulfonate, iodide, isobutyrate, lactate, maleate, malonate, methanesulfonate, mandelate metaphosphate, methanesulfonate, methoxybenzoate, methylbenzoate, monohydrogenphosphate, l-napthalenesulfonate, 2-napthalenesulfonate, nicotinate, nitrate, palmoate, pectinate, persulfate, 3-phenylpropionate, phosphate, picrate, pivalate, propionate, pyrosulfate, pyrophosphate, propiolate, phthalate, phenylacetate, phenylbutyrate, propanesulfonate, salicylate, succinate, sulfate, sulfite, succinate, suberate, sebacate, sulfonate, tartrate, thiocyanate, tosylateundeconate, and xylenesulfonate.

Further, the compounds described herein can be prepared as pharmaceutically acceptable salts formed by reacting the free base form of the compound with a pharmaceutically acceptable inorganic or organic acid, including, but not limited to, inorganic acids such as hydrochloric acid, hydrobromic acid, sulfuric acid, nitric acid, phosphoric acid metaphosphoric acid, and the like; and organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, p-toluenesulfonic acid, tartaric acid, trifluoroacetic acid, citric acid, benzoic acid, 3-(4-hydroxybenzoyl)benzoic acid, cinnamic acid, mandelic acid, arylsulfonic acid, methanesulfonic acid, ethanesulfonic acid, l,2-ethanedisulfonic acid, 2- hydroxyethanesulfonic acid, benzenesulfonic acid, 2 -naphthalene sulfonic acid, 4-methylbicyclo- [2.2.2]oct-2-ene-l -carboxylic acid, glucoheptonic acid, 4,4'-methylenebis-(3-hydroxy-2-ene-l- carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, and muconic acid.

In some embodiments, those compounds described herein which comprise a free acid group react with a suitable base, such as the hydroxide, carbonate, bicarbonate, sulfate, of a pharmaceutically acceptable metal cation, with ammonia, or with a pharmaceutically acceptable organic primary, secondary, tertiary, or quaternary amine. Representative salts include the alkali or alkaline earth salts, like lithium, sodium, potassium, calcium, and magnesium, and aluminum salts and the like. Illustrative examples of bases include sodium hydroxide, potassium hydroxide, choline hydroxide, sodium carbonate, N+(CI-4 alkyl)4, and the like.

Representative organic amines useful for the formation of base addition salts include ethylamine, diethylamine, ethylenediamine, ethanolamine, diethanolamine, piperazine, and the like. It should be understood that the compounds described herein also include the quatemization of any basic nitrogen-containing groups they contain. In some embodiments, water or oil-soluble or dispersible products are obtained by such quatemization.

### Pharmaceutical Compositions and Methods of Treatment

In some aspects, the present disclosure provides a pharmaceutical composition comprising a compound or compounds of formula (V) or (V-a) or a salt thereof as an active ingredient. In some embodiments, the present disclosure provides a pharmaceutical composition comprising a compound or compounds of formula (V) or (V-a), or pharmaceutically acceptable salts or solvates thereof, and one or more pharmaceutically acceptable carriers or excipients.

In some aspects, the present disclosure provides a pharmaceutical composition comprising a compound or compounds of formula (V) or (V-a) or a salt thereof being prepared by a method described herein, and one or more pharmaceutically acceptable carriers or excipients.

The pharmaceutical compositions of the present disclosure may be manufactured in a manner that is generally known, e.g., by means of conventional mixing, dissolving, granulating, dragee-making, levigating, emulsifying, encapsulating, entrapping, or lyophilizing processes. Pharmaceutical compositions may be formulated in a conventional manner using one or more pharmaceutically acceptable carriers comprising excipients and/or auxiliaries that facilitate processing of the active compounds into preparations that can be used pharmaceutically. Of course, the appropriate formulation is dependent upon the route of administration chosen.

In some embodiments, the pharmaceutical composition is formulated in a gel.

In some embodiments, the pharmaceutical composition is formulated in a tablet.

In some embodiments, the pharmaceutical composition is formulated in a pill.

In some embodiments, the pharmaceutical composition is formulated in a capsule.

In some embodiments, the pharmaceutical composition is formulated in a solution.

In some aspects, the present disclosure pertains, at least in part, to a compound of formula (V) or (V-a) or a salt thereof for use in a method for treating a liver disease or disorder or a lipid disease or disorder by administering to a subject in need thereof a compound of formula (V) or (V-a) or a salt thereof.

In some embodiments, the liver disease or disorder treated by these methods is fatty liver disease.

In some embodiments, the liver disease or disorder treated by these methods is nonalcoholic fatty liver disease (NAFLD). In some embodiments, the liver disease or disorder treated by these methods is NASH.

In some embodiments, the lipid disease or disorder treated by these methods is selected from the group consisting of dyslipidemia, hyperlipidemia, hypertriglyceridemia, hypercholesterolemia, low HDL, and high LDL. In some embodiments,
the hypercholesterolemia is heterozygous familial hypercholesterolemia (HeFH) or homozygous familial hypercholesterolemia (HoFH).

In some embodiments, the subject has a risk for developing the liver disease or disorder described herein. In some embodiments, the subject has a risk for developing the lipid disease or disorder described herein.

In some embodiments, the subject is a mammal. In some embodiments, the subject is a human.

### DEFINITIONS

Where a range of values is provided, it is understood that the range includes both of the endpoints with that range, as well as all intervening values.

The articles "a" and "an" as used herein and in the appended claims are used herein to refer to one or to more than one (i.e., to at least one) of the grammatical object of the article unless the context clearly indicates otherwise. By way of example, "an ultrapure form" means one ultrapure form or more than one ultrapure form.

The phrase "and/or," as used herein in the specification and in the claims, should be understood to mean "either or both". Other elements may optionally be present other than the elements specifically identified by the "and/or" clause. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements).

Unless explicitly indicated otherwise, the terms "approximately" and "about" are synonymous. In some embodiments, "approximately" and "about" refer to the recited amount, value, dose or duration ± 10%, ± 8%, ± 6%, ± 5%, ± 4%, ± 2%, ± 1%, or ± 0.5%. In some embodiments, "approximately" and "about" refer to the listed amount, value, dose, or duration ± 5%. In some embodiments, "approximately" and "about" refer to the listed amount, value, dose, or duration ± 2%. In some embodiments, "approximately" and "about" refer to the listed amount, value, dose, or duration ± 1%.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e., "one or the other but not both") when preceded by terms of exclusivity, such as "either," "one of," "only one of," or "exactly one of."

As used herein, "alkyl", "C₁, C₂, C₃, C₄, C₅ or C₆ alkyl" or "C₁-C ₆ alkyl" is intended to include C₁, C₂, C₃, C₄, C₅ or C₆ straight chain (linear) saturated aliphatic hydrocarbon groups and C₃, C₄, C₅ or C₆ branched saturated aliphatic hydrocarbon groups. For example, C₁-C ₆ alkyl is intended to include C₁, C₂, C₃, C₄, C₅ or C₆ alkyl groups. Examples of alkyl include, moieties having from one to six carbon atoms, such as, but not limited to, methyl, ethyl, n-propyl, i-propyl, n-butyl, s-butyl, t-butyl, n-pentyl, s-pentyl or n-hexyl. In certain embodiments, a straight chain or branched alkyl has six or fewer carbon atoms (e.g., C₁-C₆ for straight chain, C₃-C₆ for branched chain), and in another embodiment, a straight chain or branched alkyl has four or fewer carbon atoms.

As used herein, the term "alkenyl" includes unsaturated aliphatic groups analogous in length and possible substitution to the alkyls described above, but that contain at least one double bond. For example, the term "alkenyl" includes straight chain alkenyl groups (*e.g.,* ethenyl, propenyl, butenyl, pentenyl, hexenyl, heptenyl, octenyl, nonenyl, decenyl), and branched alkenyl groups. In certain embodiments, a straight chain or branched alkenyl group has six or fewer carbon atoms in its backbone (e.g., C₂-C₆ for straight chain, C₃-C₆ for branched chain). The term "C₂-C₆" includes alkenyl groups containing two to six carbon atoms. The term "C₃-C₆" includes alkenyl groups containing three to six carbon atoms.

As used herein, the term "alkynyl" includes unsaturated aliphatic groups analogous in length and possible substitution to the alkyls described above, but which contain at least one triple bond. For example, "alkynyl" includes straight chain alkynyl groups (e.g., ethynyl, propynyl, butynyl, pentynyl, hexynyl, heptynyl, octynyl, nonynyl, decynyl), and branched alkynyl groups. In certain embodiments, a straight chain or branched alkynyl group has six or fewer carbon atoms in its backbone (*e.g*., C₂-C₆ for straight chain, C₃-C₆ for branched chain). The term "C₂-C₆" includes alkynyl groups containing two to six carbon atoms. The term "C₃-C₆" includes alkynyl groups containing three to six carbon atoms. As used herein, "C₂-C₆ alkenylene linker" or "C₂-C₆ alkynylene linker" is intended to include C₂, C₃, C₄, C₅ or C₆ chain (linear or branched) divalent unsaturated aliphatic hydrocarbon groups. For example, C₂-C₆ alkenylene linker is intended to include C₂, C₃, C₄, C₅ and C₆ alkenylene linker groups.

"Aminoalkyl" means an alkyl moiety as defined herein, substituted with one or more amino groups.

As used herein, the term "aryl" includes groups with aromaticity, including "conjugated," or multicyclic systems with one or more aromatic rings and do not contain any heteroatom in the ring structure. The term aryl includes both monovalent species and divalent species. Examples of aryl groups include, but are not limited to, phenyl, biphenyl, naphthyl and the like. Conveniently, an aryl is phenyl.

As used herein, the term "contact" or "contacting" refers an action causing two or more reactants to be in a proximity, e.g., such that the two or more reactants chemically react. In some embodiments, the contacting comprising mixing the two or more reactants. In some embodiments, the contacting is under a reaction condition suitable for forming the desired reaction product from the two or more reactants.

As used herein, the term "cycloalkyl" refers to a saturated or unsaturated nonaromatic hydrocarbon mono- or multi-ring (e.g., fused, bridged, or spiro rings) system having 3 to 30 carbon atoms (e.g., C₃-C₁₂, C₃-C₁₀, or C₃-C₈). Examples of cycloalkyl include, but are not limited to, cyclopropyl, cyclobutyl, cyclopentyl, cyclohexyl, cycloheptyl, cyclooctyl, cyclopentenyl, cyclohexenyl, cycloheptenyl, 1,2,3,4-tetrahydronaphthalenyl, and adamantyl.

"Deuteroalkyl" refers to an alkyl group where one or more hydrogen atoms of an alkyl are replaced with deuterium.

As used herein, the term "halo" or "halogen" refers to fluoro, chloro, bromo and iodo.

"Haloalkyl" refers to an alkyl group where one or more hydrogen atoms of an alkyl are replaced with a halogen.

As used herein, the term "heterocycloalkyl" refers to a saturated or unsaturated nonaromatic 3-8 membered monocyclic, 7-12 membered bicyclic (fused, bridged, or spiro rings), or 11-14 membered tricyclic ring system (fused, bridged, or spiro rings) having one or more heteroatoms (such as O, N, S, P, or Se), e.g., 1 or 1-2 or 1-3 or 1-4 or 1-5 or 1-6 heteroatoms, or *e.g.,* 1, 2, 3, 4, 5, or 6 heteroatoms, independently selected from the group consisting of nitrogen, oxygen and sulfur, unless specified otherwise. Examples of heterocycloalkyl groups include, but are not limited to, piperidinyl, piperazinyl, pyrrolidinyl, dioxanyl, tetrahydrofuranyl, isoindolinyl, indolinyl, imidazolidinyl, pyrazolidinyl, oxazolidinyl, isoxazolidinyl, triazolidinyl, oxiranyl, azetidinyl, oxetanyl, thietanyl, 1,2,3,6-tetrahydropyridinyl, tetrahydropyranyl, dihydropyranyl, pyranyl, morpholinyl, tetrahydrothiopyranyl, 1,4-diazepanyl, 1,4-oxazepanyl, 2-oxa-5-azabicyclo[2.2.1]heptanyl, 2,5-diazabicyclo[2.2.1]heptanyl, 2-oxa-6-azaspiro[3.3]heptanyl, 2,6-diazaspiro[3.3]heptanyl, 1,4-dioxa-8-azaspiro[4.5]decanyl, 1,4-dioxaspiro[4.5]decanyl, 1-oxaspiro[4.5]decanyl, 1-azaspiro[4.5]decanyl, 3'H-spiro[cyclohexane-1,1'-isobenzofuran]-yl, 7'H-spiro[cyclohexane-1,5'-furo[3,4-b]pyridin]-yl, 3'H-spiro[cyclohexane-1,1'-furo[3,4-c]pyridin]-yl, 3-azabicyclo[3.1.0]hexanyl, 3-azabicyclo[3.1.0]hexan-3-yl, 1,4,5,6-tetrahydropyrrolo[3,4-c]pyrazolyl, 3,4,5,6,7,8-hexahydropyrido[4,3-d]pyrimidinyl, 4,5,6,7-tetrahydro-1H-pyrazolo[3,4-c]pyridinyl, 5,6,7,8-tetrahydropyrido[4,3-d]pyrimidinyl, 2-azaspiro[3.3]heptanyl, 2-methyl-2-azaspiro[3.3]heptanyl, 2-azaspiro[3.5]nonanyl, 2-methyl-2-azaspiro[3.5]nonanyl, 2-azaspiro[4.5]decanyl, 2-methyl-2-azaspiro[4.5]decanyl, 2-oxa-azaspiro[3.4]octanyl, 2-oxa-azaspiro[3.4]octan-6-yl, and the like. In the case of multicyclic non-aromatic rings, only one of the rings needs to be non-aromatic (e.g., 1,2,3,4-tetrahydronaphthalenyl or 2,3-dihydroindole).

As used herein, the term "heteroaryl" is intended to include a stable 5-, 6-, or 7-membered monocyclic or 7-, 8-, 9-, 10-, 11- or 12-membered bicyclic aromatic heterocyclic ring which consists of carbon atoms and one or more heteroatoms, e.g., 1 or 1-2 or 1-3 or 1-4 or 1-5 or 1-6 heteroatoms, or *e.g.,* 1, 2, 3, 4, 5, or 6 heteroatoms, independently selected from the group consisting of nitrogen, oxygen and sulfur. The nitrogen atom may be substituted or unsubstituted (*i.e.,* N or NR wherein R is H or other substituents, as defined). The nitrogen and sulfur heteroatoms may optionally be oxidized (*i.e.,* N→O and S(O)ₚ, where p = 1 or 2). It is to be noted that total number of S and O atoms in the aromatic heterocycle is not more than 1. Examples of heteroaryl groups include pyrrole, furan, thiophene, thiazole, isothiazole, imidazole, triazole, tetrazole, pyrazole, oxazole, isoxazole, pyridine, pyrazine, pyridazine, pyrimidine, and the like.

"Hydroxyalkyl" means an alkyl moiety as defined herein, substituted with one or more hydroxy groups. Representative examples include, but are not limited to, hydroxymethyl, 2-hydroxyethyl, 2-hydroxypropyl, 3-hydroxypropyl, 1-(hydroxymethyl)-2-methylpropyl, 2-hydroxybutyl, 3-hydroxybutyl, 4-hydroxybutyl, 2,3-dihydroxypropyl, 2-hydroxy-1-hydroxymethylethyl, 2,3-dihydroxybutyl, 3,4-dihydroxybutyl and 2-(hydroxymethyl)-3-hydroxypropyl.

It is understood that the terms "aryl" and "heteroaryl" include multicyclic aryl and heteroaryl groups, *e.g.,* tricyclic, bicyclic, *e.g.,* naphthalene, benzoxazole, benzodioxazole, benzothiazole, benzoimidazole, benzothiophene, quinoline, isoquinoline, naphthrydine, indole, benzofuran, purine, benzofuran, deazapurine, indolizine.

As used herein, the term "pharmaceutically acceptable" refers to those compounds, anions, cations, materials, compositions, carriers, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, the term "subject" is interchangeable with the term "subject in need thereof," both of which refer to a subject having a disease or having an increased risk of developing the disease. A "subject" includes a mammal. The mammal can be *e.g.,* a human or appropriate non-human mammal, such as primate, mouse, rat, dog, cat, cow, horse, goat, camel, sheep or a pig. In some embodiments, the mammal is a human.

As used herein, the term "tautomer" is one of two or more structural isomers that exist in equilibrium and is readily converted from one isomeric form to another. This conversion results in the formal migration of a hydrogen atom accompanied by a switch of adjacent conjugated double bonds. Tautomers exist as a mixture of a tautomeric set in solution. In solutions where tautomerisation is possible, a chemical equilibrium of the tautomers will be reached. The exact ratio of the tautomers depends on several factors, including temperature, solvent and pH. The concept of tautomers that are interconvertible by tautomerisations is called tautomerism. Of the various types of tautomerism that are possible, two are commonly observed. In keto-enol tautomerism a simultaneous shift of electrons and a hydrogen atom occurs. Ring-chain tautomerism arises as a result of the aldehyde group (-CHO) in a sugar chain molecule reacting with one of the hydroxy groups (-OH) in the same molecule to give it a cyclic (ring-shaped) form as exhibited by glucose.

It is to be understood that the compounds of the present disclosure may be depicted as different tautomers. It should also be understood that when compounds have tautomeric forms, all tautomeric forms are intended to be included in the scope of the present disclosure, and the naming of the compounds does not exclude any tautomer form. It will be understood that certain tautomers may have a higher level of activity than others.

As used herein, the term "treating" or "treat" describes the management and care of a patient for the purpose of combating a disease, condition, or disorder and includes the administration of a compound of the present disclosure, or a pharmaceutically acceptable salt, polymorph or solvate thereof, to alleviate the symptoms or complications of a disease, condition or disorder, or to eliminate the disease, condition or disorder. The term "treat" can also include treatment of a cell *in vitro* or an animal model.

As used herein, the term "salt" or "pharmaceutically acceptable salt" refers to a derivative of the compounds of the present disclosure wherein the parent compound is modified by making acid or base salts thereof. Examples of pharmaceutically acceptable salts include, but are not limited to, mineral or organic acid salts of basic residues such as amines, alkali or organic salts of acidic residues such as carboxylic acids, and the like. The pharmaceutically acceptable salts include the conventional non-toxic salts or the quaternary ammonium salts of the parent compound formed, for example, from non-toxic inorganic or organic acids. For example, such conventional non-toxic salts include, but are not limited to, those derived from inorganic and organic acids selected from 2-acetoxybenzoic, 2-hydroxyethane sulfonic, acetic, ascorbic, benzene sulfonic, benzoic, bicarbonic, carbonic, citric, edetic, ethane disulfonic, 1,2-ethane sulfonic, fumaric, glucoheptonic, gluconic, glutamic, glycolic, glycollyarsanilic, hexylresorcinic, hydrabamic, hydrobromic, hydrochloric, hydroiodic, hydroxymaleic, hydroxynaphthoic, isethionic, lactic, lactobionic, lauryl sulfonic, maleic, malic, mandelic, methane sulfonic, napsylic, nitric, oxalic, pamoic, pantothenic, phenylacetic, phosphoric, polygalacturonic, propionic, salicylic, stearic, subacetic, succinic, sulfamic, sulfanilic, sulfuric, tannic, tartaric, toluene sulfonic, and the commonly occurring amine acids, e.g., glycine, alanine, phenylalanine, arginine, etc. Other examples of pharmaceutically acceptable salts include hexanoic acid, cyclopentane propionic acid, pyruvic acid, malonic acid, 3-(4-hydroxybenzoyl)benzoic acid, cinnamic acid, 4-chlorobenzenesulfonic acid, 2-naphthalenesulfonic acid, 4-toluenesulfonic acid, camphorsulfonic acid, 4-methylbicyclo-[2.2.2]-oct-2-ene-1-carboxylic acid, 3-phenylpropionic acid, trimethylacetic acid, tertiary butylacetic acid, muconic acid, and the like. The present disclosure also encompasses salts formed when an acidic proton present in the parent compound either is replaced by a metal ion, *e.g.,* an alkali metal ion, an alkaline earth ion, or an aluminum ion; or coordinates with an organic base such as ethanolamine, diethanolamine, triethanolamine, tromethamine, N-methylglucamine, and the like. In the salt form, it is understood that the ratio of the compound to the cation or anion of the salt can be 1:1, or any ratio other than 1:1, e.g., 3:1, 2:1, 1:2, or 1:3. It is to be understood that all references to pharmaceutically acceptable salts include solvent addition forms (solvates) or crystal forms (polymorphs) as defined herein, of the same salt.

All percentages and ratios used herein, unless otherwise indicated, are by weight. Other features and advantages of the present disclosure are apparent from the different examples. The provided examples illustrate different components and methodology useful in practicing the present disclosure.

As used herein, the term "MGL-3196" is equivalent to 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile (i.e., 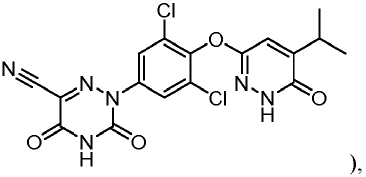 or any of its pharmaceutically acceptable salts.

Citation of publications and patent documents is not intended as an admission that any is pertinent prior art, nor does it constitute any admission as to the contents or date of the same. The invention having now been described by way of written description, those of skill in the art will recognize that the invention can be practiced in a variety of embodiments and that the foregoing description and examples below are for purposes of illustration and not limitation of the claims that follow.

### EXAMPLES

Unless otherwise specified, the analytical instruments and parameters used for compounds described in the Examples are as follows:

Nuclear magnetic resonance (NMR) spectra were recorded on a Bruker NEO 600 MHz NMR spectrometer equipped with a 5 mm broadband observe probe. For ¹H NMR, 16 scans were co-added with a 90 degree pulse and a recycle delay of 10 seconds. For ¹³C NMR, ¹³C detection was accompanied with a ¹H composite pulse decoupling. Unless otherwise noted, 256 scans were co-added for ¹³C NMR data collection. The chemical shift (δ) is reported in parts per million (ppm).

LC-MS chromatograms and spectra were recorded using a Shimadzu LCMS-2020 ultra high-speed mass spectrometer interfaced with a Shimadzu LC-2040C 3D liquid chromatography system.

The XRPD data were collected using a Rigaku X-ray generator: 30kV, 15 mA; Wavelength: K alpha 1, Goniometry: MiniFlex goniometer, Scan speed: 2.0000 °/min, Scan step: 0.02 °, Detector: Miniflex counter, Scan range: 3.0000 - 45.0000.

Melting points were acquired using a TA Instruments Inc. DSC Q200.

### Abbreviations:

- ACN and MeCN: acetonitrile
- CDCl₃: chloroform-d
- DCM: dichloromethane
- DMF: N,N-dimethylformamide
- g: gram(s)
- HPLC: high performance liquid chromatography
- mL: milliliter(s)
- MHz: mega hertz
- THF: tetrahydrofuran

### Example 1: Synthesis of 6-(4-Amino-2,6-dichloro-phenoxy)-4-isopropyl-2H-pyridazin-3-one (Int. 7)

### Synthesis of 6-(2, 6-Dichloro-4-nitro-phenoxy)-4-isopropyl-2H-pyridazin-3-one (compound 1-4)

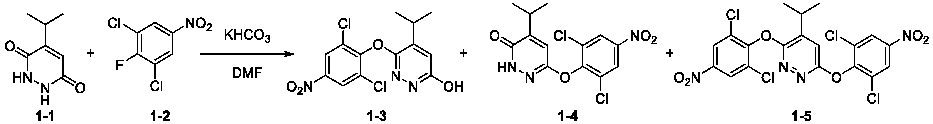

Potassium bicarbonate (2.34 g, 23.4 mmol) was added to the solution of compounds 3-1 (3.0 g, 19.5 mmol) and 3-2 (2.9 g, 13.6 mmol) in DMF (60 mL) and the mixture was stirred at room temperature for two hours before the reaction was quenched by water (180 mL). The resulting suspension was filtered. The filter cake was washed with water (60 mL) and dried under vacuum for 24 hours to afford crude compound 1-4 (4.93 g), which was further purified by slurry in ethyl acetate to afford compound 1-4 as an off-white solid (3.51 g, 74.9% purity). Compound 1-3, the regio-isomer of compound 1-4, was obtained by column chromatography purification.

Compound 1-4: ¹H NMR (600 MHz, CDCl₃) δ 8.31 (s, 2H), 7.16 (d, J = 0.8 Hz, 1H), 3.24 (m, 1H), 1.30 (d, J = 6.9 Hz, 3H). ¹³C NMR (150 MHz, CDCl₃) δ 160.72, 154.89, 151.70, 150.85, 145.18, 130.55, 124.31, 119.65, 28.22, 20.75. LRMS for C₁₃H₁₂Cl₂N₃O₄ [M+H+] m/z = 344, C₁₃H₁₀Cl₂N₃O₄ [M+H-] m/z = 342. Crystalline solid of compound 1-4 was obtained from recrystallization in a solvent mixture of DCM and THF. Melting point: 270.82 °C. The peak values of the XRPD of compound 1-4 are listed below (PEAK: 21-pts/Parabolic Filter, Threshold=3.0, Cutoff=0.1%, BG=3/0.6, Peak-Top=Summit).

| 2-Theta | d(A) |
|---|---|
| 3.82 | 23.1098 |
| 4.079 | 21.6467 |
| 11.06 | 7.9932 |
| 14.68 | 6.0292 |
| 18.941 | 4.6814 |
| 20.12 | 4.4096 |
| 21.559 | 4.1184 |
| 21.96 | 4.0443 |
| 22.699 | 3.9142 |
| 24 | 3.7049 |
| 24.48 | 3.6333 |
| 24.8 | 3.5871 |
| 25.481 | 3.4928 |
| 26.559 | 3.3533 |
| 29.179 | 3.0579 |
| 31.28 | 2.8572 |
| 31.661 | 2.8237 |
| 32.781 | 2.7297 |
| 33.08 | 2.7057 |
| 34.538 | 2.5947 |
| 34.959 | 2.5645 |
| 35.42 | 2.5322 |
| 38.101 | 2.3599 |
| 39.98 | 2.2532 |
| 40.36 | 2.2329 |
| 41.901 | 2.1543 |
| 44.501 | 2.0343 |

Compound 1-3 (pure by HPLC): ¹H NMR (600 MHz, CDCl₃) δ 10.76 (s, 1H, NH), 8.27 (s, 2H), 6.84 (s, 1H), 3.16 (m, 1H), 1.34 (d, J = 6.8 Hz, 6H). ¹³C NMR (150 MHz, CDCl₃) δ 161.98, 151.08, 150.64, 147.85, 145.42, 130.64, 128.36, 124.50, 28.63, 21.19. LRMS for C₁₃H₁₂Cl₂N₃O₄ [M+H+] m/z = 344.

### Synthesis of 6-(4-Amino-2,6-dichloro-phenoxy)-4-isopropyl-2H-pyridazin-3-one (Int. 7)

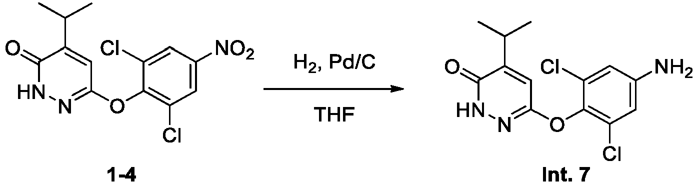

Palladium on carbon (5%, 50 mg) was added to the solution of compound 1-4 (0.5 g, 1.5 mmol) in THF (25 mL). The solution was purged with nitrogen twice and then stirred under hydrogen atmosphere at room temperature for 5 hours. The mixture was filtered through a celite pad and the filtrate was concentrated to dryness to afford Int. 7 as an off-white solid (473 mg, 103.6%). ¹H NMR (600 MHz, CDCl₃) δ 7.05 (s, 1H), 6.64 (s, 2H), 3.18 (m, 1H), 1.24 (d, J = 6.9 Hz, 6H). ¹³C NMR (151 MHz, CDCl₃) δ 160.53, 154.12, 152.89, 145.35, 137.15, 129.41, 120.44, 114.87, 28.29, 21.00. LRMS for C₁₃H₁₁Cl₂N₃O₂ [M+H-] m/z = 312.

### Example 2. 6-(4-amino-2,6-dichlorophenoxy)-4,5-dimethylpyridazin-3(2H)-one (2-3)

### Synthesis of 6-(2,6-dichloro-4-nitrophenoxy)-4,5-dimethylpyridazin-3(2H)-one (2-2)

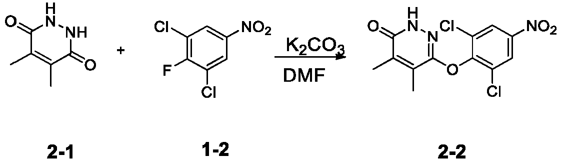

Potassium carbonate (118 mg, 0.86 mmol) was added to the solution of 4,5-dimethyl-1,2-dihydropyridazine-3,6-dione (compound 2-1, 80 mg, 0.57 mmol) and compound 1-2 (84 mg, 0.40 mmol) in DMF (2.0 mL) and the mixture was stirred at room temperature for 3 hours before the reaction was quenched with water (6.0 mL). The resulting suspension was filtered. The filter cake was washed with water (2.0 mL) and dried under vacuum for 24 hours to afford crude compound 2-2 (114 mg), which was further purified by slurry in ethyl acetate to afford compound 2-2 as an off-white amorphous solid (93 mg, yield: 49.5%). Compound 2-2: ¹H NMR (600 MHz, CDCl₃) δ 10.72 (s, 1H, NH), 8.31 (s, 2H), 2.38 (s, 3H), 2.26 (s, 3H). ¹³C NMR (151 MHz, CDCl₃) δ 161.71, 151.52, 151.27, 145.28, 140.78, 133.08, 130.66, 124.47, 13.36, 13.00. LRMS for C₁₃H₁₀C₁₂N₃O₄ [M+H+] m/z = 330.

### Synthesis of 6-(4-amino-2,6-dichlorophenoxy)-4,5-dimethylpyridazin-3(2H)-one (2-3)

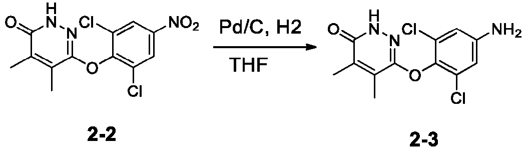

Palladium on carbon (5%, 3.0 mg) was added to the solution of compound 2-2 (30 mg, 0.09 mmol) in THF (2.0 mL). The solution was purged with nitrogen twice and then stirred under hydrogen atmosphere at room temperature for 5 hours. The mixture was filtered through a celite pad and the filtrate was concentrated to dryness to afford compound 2-3 as an off-white solid (32 mg, 116%). Compound 2-3: ¹H NMR (600 MHz, CDCl₃) δ 6.69 (s, 2H), 2.34 (s, 3H), 2.20 (s, 3H). ¹³C NMR (151 MHz, CDCl₃) δ 163.28, 153.70, 148.80, 140.39, 137.37, 135.21, 130.01, 115.16, 13.25, 12.65. LRMS for C₁₂H₁₂Cl₂N₃O₂ [M+H+] m/z = 300.

### Example 3. Synthesis of 6-(4-amino-2,6-dichlorophenoxy)-4-methylpyridazin-3(2H)-one (3-4) and 6-(4-amino-2,6-dichlorophenoxy)-5-methylpyridazin-3(2H)-one (3-5)

### 6-(2,6-dichloro-4-nitrophenoxy)-4-methylpyridazin-3(2H)-one (3-2) and 6-(2, 6-dichloro-4-nitrophenoxy)-5-methylpyridazin-3(2H)-one (3-3)

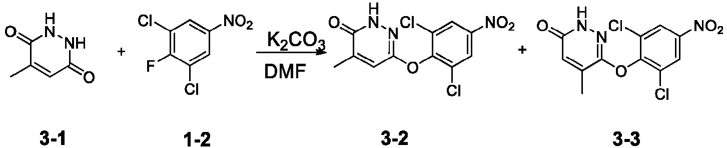

Potassium carbonate (329 mg, 2.38 mmol) was added to the solution of 4-methyl-1,2-dihydropyridazine-3,6-dione (compound 3-1, 200 mg, 1.59 mmol) and compound 1-2 (233 mg, 1.11 mmol) in DMF (4.0 mL) and the mixture was stirred at room temperature for 3 hours before the reaction was quenched with water (12.0 mL). The resulting suspension was filtered. The filter cake was washed with water (4.0 mL) and dried under vacuum for 24 hours to afford crude compound 3-2/3-3 (310 mg). Further purification by column chromatography gave off-white crystalline solid compound 3-2 (139 mg, yield: 27.8%) and off-white amorphous solid 3-3 (74 mg, yield: 14.8%). Compound 3-2: ¹H NMR (600 MHz, DMSO-*d₆*) δ 12.29 (s, 1H, NH), 8.51 (s, 2H), 7.60 (s, 1H), 2.14 (s, 3H). ¹³C NMR (151 MHz, DMSO-*d₆*,) δ 160.50, 150.44, 150.12, 145.35, 145.33, 129.31, 124.67, 122.37, 16.24. LRMS for C₁₁H₈Cl₂N₃O₂ [M+H+] m/z = 316. Compound 3-3: ¹H NMR (600 MHz, DMSO-*d₆*) δ 12.23 (s, 1H, NH), 8.53 (s, 2H), 6.99 (s, 1H), 2.31 (s, 3H). ¹³C NMR (151 MHz, DMSO-*d₆*) δ 160.07, 150.45, 150.29, 145.36, 136.98, 131.82, 129.15, 124.67, 15.61. LRMS for C₁₁H₈Cl₂N₃O₂ [M+H+] m/z = 316.

### Synthesis of 6-(4-amino-2,6-dichlorophenoxy)-4-methylpyridazin-3(2H)-one (3-4)

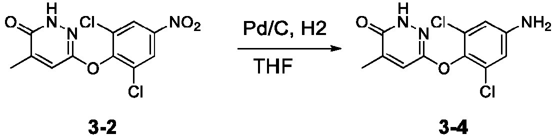

Palladium on carbon (5%, 2.5 mg) was added to the solution of compound 3-2 (25 mg, 0.08 mmol) in THF (2.0 mL). The solution was purged with nitrogen twice and then stirred under hydrogen atmosphere at room temperature for 5 hours. The mixture was filtered through a celite pad and the filtrate was concentrated to dryness to afford compound 3-4 as an off-white solid (21 mg, yield: 92.9%). Compound 3-4: ¹H NMR (600 MHz, DMSO-*d₆*) δ 12.11 (s, 1H, NH), 7.40 (s, 1H), 6.66 (s, 2H), 2.09 (s, 3H). ¹³C NMR (151 MHz, DMSO-*d₆*) δ 160.56, 151.37, 147.96, 144.14, 133.78, 127.84, 122.72, 112.88, 16.11. LRMS for C₁₁H₁₀C₁₂N₃O₂ [M+H+] m/z = 286.

### Synthesis of 6-(4-amino-2,6-dichlorophenoxy)-5-methylpyridazin-3(2H)-one (3-5)

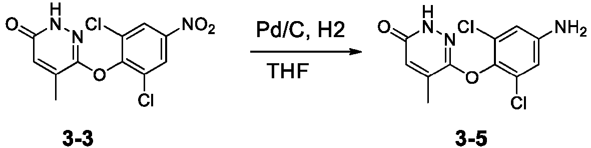

Palladium on carbon (5%, 1.5 mg) was added to the solution of compound 3-3 (15 mg, 0.05 mmol) in THF (5.0 mL). The solution was purged with nitrogen twice and then stirred under hydrogen atmosphere at room temperature for 5 hours. The mixture was filtered through a celite pad and the filtrate was concentrated to dryness to afford compound 3-5 as an off-white solid (16 mg, yield: 107%). Compound 3-5: ¹H NMR (600 MHz, CD₃OD) δ 6.89 (s, 1H), 6.70 (s, 2H), 2.35 (s, 3H). ¹³C NMR (151 MHz, CD₃OD) δ 163.52, 154.04, 148.90, 140.86, 137.19, 131.34, 129.90, 115.13, 16.60. LRMS for C₁₁H₁₀Cₗ₂N₃O₂ [M+H+] m/z = 286.

### Example 4. Synthesis of 6-(4-amino-2,6-dichlorophenoxy)-4-phenylpyridazin-3(2H)-one (4-3)

### Synthesis of 6-(2, 6-dichloro-4-nitrophenoxy)-4-phenylpyridazin-3(2H)-one (4-2)

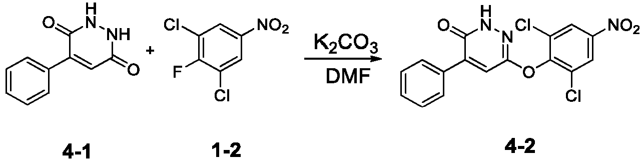

Potassium carbonate (249 mg, 1.80 mmol) was added to the solution of 4-phenyl-1,2-dihydropyridazine-3,6-dione (compound 4-1, 226 mg, 1.20 mmol) and compound 1-2 (177 mg, 0.84 mmol) in DMF (5.0 mL) and the mixture was stirred at room temperature for 3 hours before the reaction was quenched with water (20 mL). The resulting suspension was filtered. The filter cake was washed with water (5.0 mL) and dried under vacuum for 24 hours to afford crude compound 4-2 (177 mg), which was further purified by slurry in ethyl acetate to afford compound 4-2 as a light brown crystalline solid (110 mg, yield: 24.2%). Compound 4-2: ¹H NMR (600 MHz, CDCl₃) δ 11.02 (s, 1H, NH), 8.27 (m, 2H), 7.84 (m, 2H), 7.48 (m 3H), 7.43 (s, 1H). ¹³C NMR (151 MHz, CDCl₃) δ 160.66, 152.04, 151.01, 145.45, 144.52, 132.91, 130.77, 128.94, 128.92, 128.82, 124.56, 122.07. LRMS for C₁₆H₁₀Cl₂N₃O₂ [M+H⁺] m/z = 378.

### Synthesis of 6-(4-amino-2,6-dichlorophenoxy)-4-phenylpyridazin-3(2H)-one (4-3)

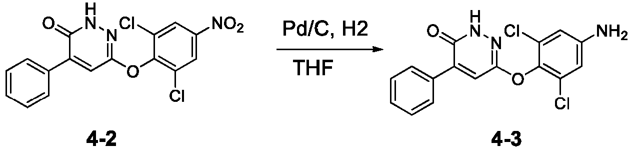

Palladium on carbon (5%, 3.0 mg) was added to the solution of compound 4-2 (30 mg, 0.08 mmol) in THF (2.0 mL). The solution was purged with nitrogen twice and then stirred under hydrogen atmosphere at room temperature for 5 hours. The mixture was filtered through a celite pad and the filtrate was concentrated to dryness to afford compound 4-3 as a light brown solid (23 mg, yield: 83.3%). Compound 4-3: ¹H NMR (600 MHz, DMSO-*d*₆) δ 7.85 - 7.84 (m, 2H), 7.56 (s, 1H), 7.50-7.48 (m, 3H), 6.71 (s, 2H). ¹³C NMR (151 MHz, DMSO-*d*₆) δ 152.41, 145.26, 139.33, 135.14, 127.44, 125.18, 121.47, 120.54, 120.21, 119.99, 114.46, 105.52. LRMS for C₁₆H₁₂C₁₂N₃O₂ [M+H+] m/z = 348.

### Example 5. Synthesis of 6-(4-amino-2,6-dichlorophenoxy)-4-(trifluoromethyl)pyridazin-3(2H)-one (5-3)

### Synthesis of 6-(2, 6-dichloro-4-nitrophenoxy)-4-(trifluoromethyl)pyridazin-3(2H)-one (5-2)

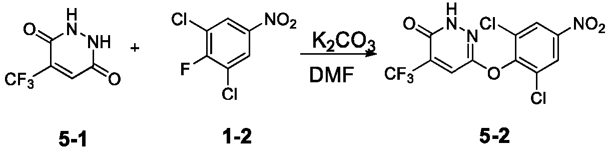

Sodium bicarbonate (46.7 mg, 0.56 mmol) was added to the solution of 4-(trifluoromethyl)-1,2-dihydropyridazine-3,6-dione (compound 5-1, 100 mg, 0.56 mmol) and compound 1-2 (58.3 mg, 0.28 mmol) in DMF (2.0 mL) below 5.0 °C, and the mixture was stirred at room temperature for 4 days before the reaction was quenched with water (6.0 mL). The resulting suspension was filtered. The filter cake was washed with water (2.0 mL) and dried under vacuum for 24 hours to afford crude compound 5-2 (109 mg), which was further purified by slurry in ethyl acetate to afford compound 5-2 as an off-white solid (58 mg, yield: 28.3%). Compound 5-2: ¹H NMR (600 MHz, DMSO-*d*₆) δ 13.11 (s, 1H, NH), 8.55 (s, 2H), 8.38 (s, 1H). LRMS for C₁₁H₅Cl₂F₃N₃O₄ [M+H⁺] m/z = 370).

### Synthesis of 6-(4-amino-2,6-dichlorophenoxy)-4-(trifluoromethyl)pyridazin-3(2H)-one (5-3)

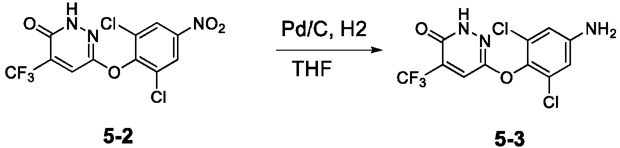

Palladium on carbon (5%, 1.5 mg) was added to the solution of compound 5-2 (15 mg, 0.04 mmol) in THF (2.0 mL). The solution was purged with nitrogen twice and then stirred under hydrogen atmosphere at room temperature for 5 hours. The mixture was filtered through a celite pad and the filtrate was concentrated to dryness. Compound 5-3 was obtained as an off-white solid after column chromatography purification (6.1 mg, yield: 44.3%). Compound 5-3: ¹H NMR (600 MHz, CDCl₃) δ 10.02 (s, 1H, NH), 7.60 (s, 1H), 6.63 (s, 2H). LRMS for C₁₁H₇Cl₂F₃N₃O₂ [M+H⁺] m/z = 340.

### Example 6. Synthesis of 4-(4-amino-2,6-dichlorophenoxy)-5,6,7,8-tetrahydrophthalazin-1(2H)-one (6-3)

### Synthesis of 4-(2, 6-dichloro-4-nitrophenoxy)-5, 6,7,8-tetrahydrophthalazin-1(2H)-one (6-2)

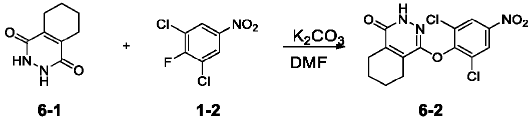

Potassium carbonate (699 mg, 5.05 mmol) was added to the solution of 2,3,5,6,7,8-hexahydrophthalazine-1,4-dione (compound 6-1, 560 mg, 3.37 mmol) and compound 1-2 (495 mg, 2.36 mmol) in DMF (5.0 mL) and the mixture was stirred at room temperature for 3 hours before the reaction was quenched with water (20 mL). The resulting suspension was filtered. The filter cake was washed with water (5.0 mL) and dried under vacuum for 24 hours to afford crude compound 6-2 (821 mg), which was further purified by slurry in ethyl acetate to afford compound 6-2 as a white solid (722 mg, yield: 60.2%). Compound 6-2: ¹H NMR (600 MHz, DMSO-*d*₆) δ 12.15 (s, 1H, NH), 8.51 (s, 2H), 2.65 (t, *J =* 5.8 Hz, 2H), 2.44 (t, *J =* 5.8 Hz, 2H), 1.81 - 1.70 (m, 4H). ¹³C NMR (151 MHz, DMSO-*d*₆) δ 159.90, 150.59, 149.67, 145.28, 141.37, 132.70, 129.28, 124.61, 23.03, 22.53, 20.26, 20.13. LRMS for C₁₄H₁₂Cl₂N₃O₄ [M+H⁺] m/z = 356.

### Synthesis of 4-(4-amino-2,6-dichlorophenoxy)-5,6,7,8-tetrahydrophthalazin-1(2H)-one (6-3)

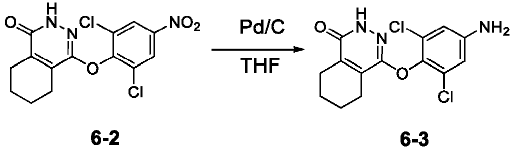

Palladium on carbon (5%, 2.5 mg) was added to the solution of compound 6-2 (25 mg, 0.07 mmol) in THF (2.0 mL). The solution was purged with nitrogen twice and then stirred under hydrogen atmosphere at room temperature for 5 hours. The mixture was filtered through a celite pad and the filtrate was concentrated to dryness to afford compound 6-3 as an off-white solid (20 mg, yield: 87.3%). Compound 6-3: ¹H NMR (600 MHz, CD₃OD) δ 6.70 (s, 2H), 2.73 (t, *J =* 4.9 Hz, 2H), 2.54 (t, *J =* 4.9 Hz, 2H), 1.88 - 1.78 (m, 4H). ¹³C NMR (151 MHz, CD₃OD) δ 163.02, 153.43, 148.52, 141.45, 137.42, 136.55, 130.03, 115.30, 31.04, 24.38, 22.03, 21.91. LRMS for C₁₄H₁₄Cl₂N₃O₂ [M+H⁺] m/z = 326.

### Example 7. Synthesis of 4-(4-amino-2,6-dichlorophenoxy)phthalazin-1(2H)-one (7-3)

### Synthesis of 4-(2, 6-dichloro-4-nitrophenoxy)phthalazin-1(2H)-one (7-2)

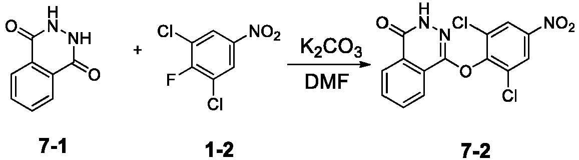

Potassium carbonate (2.60 g, 18.50 mmol) was added to the solution of phthalic hydrazide (compound 7-1, 2.00 g, 12.33 mmol) and compound 1-2 (1.81 g, 8.63 mmol) in DMF (20 mL) and the mixture was stirred at room temperature for 3 hours before the reaction was quenched with water (60 mL). The resulting suspension was filtered. The filter cake was washed with water (20 mL) and dried under vacuum for 24 hours to afford crude compound 7-2(2.98 g), which was further purified by slurry in ethyl acetate to afford compound 7-2 as an off-white solid (2.2 g, yield: 50.7%). Compound 7-2: ¹H NMR (600 MHz, DMSO-*d*₆) δ 12.09 (s, 1H, NH), 8.55 (s, 2H), 8.32 (d, *J =* 7.5 Hz, 1H), 8.23 (d, *J =* 7.8 Hz, 1H), 8.12 - 8.07 (m, 1H), 8.06 - 8.00 (m, 1H). ¹³C NMR (151 MHz, DMSO-*d*₆) δ 158.89, 150.39, 147.70, 145.43, 134.33, 133.40, 129.40, 129.09, 126.70, 124.63, 123.34, 122.77. LRMS for C₁₄H₈Cl₂N₃O₄ [M+H⁺] m/z = 352.

### Synthesis of 4-(4-amino-2,6-dichlorophenoxy)phthalazin-1(2H)-one (7-3)

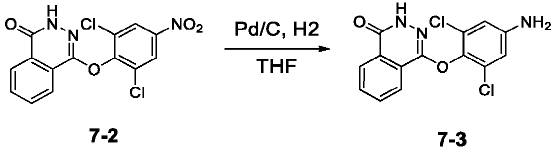

Palladium on carbon (5%, 10 mg) was added to the solution of compound 7-2 (100 mg, 0.28 mmol) in THF (10 mL). The solution was purged with nitrogen twice and then stirred under hydrogen atmosphere at room temperature for 5 hours. The mixture was filtered through a celite pad and the filtrate was concentrated to dryness to afford compound 7-3 as a light brown solid (98 mg, yield: 107%). Compound 7-3:¹H NMR (600 MHz, DMSO-*d*₆) δ 11.86 (s, 1H, NH), 8.28 (d, *J =* 7.7 Hz, 1H), 8.19 (d, *J =* 7.8 Hz, 1H), 8.05 - 8.01 (m, 1H), 8.00 - 7.95 (m, 1H), 6.71 (s, 2H), 5.64 (s, 2H, NH₂). ¹³C NMR (151 MHz, DMSO-*d*₆) δ 158.88, 148.48, 148.03, 134.00, 133.90, 132.85, 128.88, 127.90, 126.46, 123.40, 123.37, 112.92. LRMS for C₁₄H₁₀CL₂N₃O₂ [M+H⁺] m/z = 322.

### Example 8. Synthesis of 6-(4-Amino-2,6-dichloro-phenoxy)-4-isopropyl-2H-pyridazin-3-one (8-1)

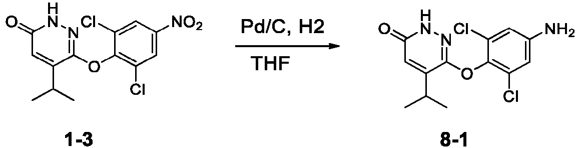

Palladium on carbon (5%, 45 mg) was added to the solution of compound 1-3 (450 mg, 1.31 mmol) in THF (25 mL). The solution was purged with nitrogen twice and then stirred under hydrogen atmosphere at room temperature for 5 hours. The mixture was filtered through a celite pad and the filtrate was concentrated to dryness to afford compound 8-1 as an off-white solid (410 mg, 99.8%). Compound 8-1: ¹H NMR (600 MHz, CDCl₃) δ 12.08 (s, NH), 6.79 (s, 1H), 6.68 (s, 2H), 3.05 (m, 1H), 1.27 (d, *J =* 6.9 Hz, 6H). ¹³C NMR (151 MHz, CDCl₃) δ 160.92, 150.65, 148.46, 146.69, 134.17, 128.21, 128.02, 113.41, 28.24, 21.16. LRMS for C₁₃H₁₃Cl₂N₃O₂ [M+H⁺] m/z = 314.

## Claims

1. A method comprising contacting a compound of formula (I), a tautomer or a salt thereof: 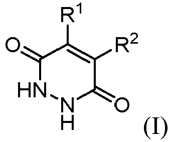 with a compound of formula (II) or a salt thereof: 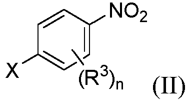 in a first organic solvent in the presence of a base, to form a compound of formula (III) or a salt thereof: 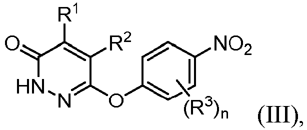 and
(A) contacting the compound of formula (III) or a salt thereof with a second organic solvent and a reducing agent to form a compound of formula (IV) or a salt thereof: 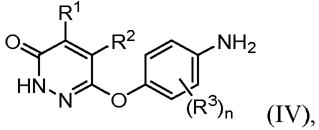 wherein:
R¹ and R² are each independently hydrogen, deuterium, halogen, -CN, -OH, -OR^{a}, - SH, -SR^{a}, -S(=O)R^{a}, -S(=O)₂R^{a}, -NO₂, -NR^{b}R^{c}, -NHS(=O)₂R^{a}, -S(=O)₂NR^{b}R^{c}, -C(=O)R^{a}, - OC(=O)R^{a}, -C(=O)OR^{b}, -OC(=O)OR^{b}, -C(=O)NR^{b}R^{c}, -OC(=O)NR^{b}R^{c}, -NR^{b}C(=O)NR^{b}R^{c}, - NR^{b}C(=O)R^{a}, -NR^{b}C(=O)OR^{b}, C₁-C₆ alkyl, C₁-C₆ deuteroalkyl, C₁-C₆ haloalkyl, C₄-C₆ hydroxyalkyl, C₁-C₆ aminoalkyl, C₂-C₆ alkenyl, C₂-C₆ alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OR^{a}, -NR^{b}R^{c}, -C(=O)R^{a}, -C(=O)OR^{b}, -C(=O)NR^{b}R^{c}, C₁-C₆ alkyl, or C₁-C₆ haloalkyl;
each R³ is independently hydrogen, deuterium, halogen, -CN, -OH, -OR^{a}, -SH, -SR^{a}, - S(=O)R^{a}, -S(=O)₂R^{a}, -NO₂, -NR^{b}R^{c}, -NHS(=O)₂R^{a}, -S(=O)₂NR^{b}R^{c}, -C(=O)R^{a}, -OC(=O)R^{a}, - C(=O)OR^{b}, -OC(=O)OR^{b}, -C(=O)NR^{b}R^{c}, -OC(=O)NR^{b}R^{c}, -NR^{b}C(=O)NR^{b}R^{c}, -NR^{b}C(=O)R^{a}, -NR^{b}C(=O)OR^{b}, C₁-C₆ alkyl, C₁-C₆ deuteroalkyl, C₁-C₆ haloalkyl, C₁-C₆ hydroxyalkyl, C₁-C₆ aminoalkyl, C₂-C₆ alkenyl, C₂-C₆ alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OR^{a}, -NR^{b}R^{c}, -C(=O)R^{a}, -C(=O)OR^{b}, -C(=O)NR^{b}R^{c}, C₁-C₆ alkyl, or C₁-C₆ haloalkyl;
each R^{a} is independently C₁-C₆ alkyl, C₁-C₆ deuteroalkyl, C₁-C₆ haloalkyl, C₁-C₆ hydroxyalkyl, C₁-C₆ aminoalkyl, C₂-C₆ alkenyl, C₂-C₆ alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OCH₃, -NH₂, -C(=O)CH₃, -C(=O)OH, -C(=O)OCH₃, C₁-C₆alkyl, or C₁-C₆ haloalkyl;
each R^{b} is independently hydrogen, deuterium, C₁-C₆ alkyl, C₁-C₆ deuteroalkyl, C₁-C₆ haloalkyl, C₁-C₆ hydroxyalkyl, C₁-C₆ aminoalkyl, C₂-C₆ alkenyl, C₂-C₆ alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OCH₃, -NH₂, -C(=O)CH₃, -C(=O)OH, - C(=O)OCH₃, C₁-C₆ alkyl, or C₁-C₆ haloalkyl;
each R^{c} is independently hydrogen, deuterium, C₁-C₆ alkyl, C₁-C₆ deuteroalkyl, C₁-C₆ haloalkyl, C₁-C₆ hydroxyalkyl, C₁-C₆ aminoalkyl, C₂-C₆ alkenyl, C₂-C₆ alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OCH₃, -NH₂, -C(=O)CH₃, -C(=O)OH, - C(=O)OCH₃, C₁-C₆ alkyl, or C₁-C₆ haloalkyl;
X is halogen; and
n is 0, 1, 2, 3, or 4, or
(B) contacting the compound of formula (III) or a salt thereof with R⁶X to form a compound of formula (III-a) or a salt thereof: 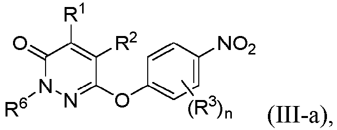 wherein:
R⁶ is -CN, -OH, -OR^{a}, -S(=O)R^{a}, -S(=O)₂R^{a}, -S(=O)₂NR^{b}R^{c}, -C(=O)R^{a}, -OC(=O)R^{a}, - C(=O)OR^{b}, -C(=O)NR^{b}R^{c}, C₁-C₆ alkyl, C₁-C₆ deuteroalkyl, C₁-C₆ haloalkyl, C₁-C₆ hydroxyalkyl, C₁-C₆ aminoalkyl, C₂-C₆ alkenyl, C₂-C₆ alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OR^{a}, -NR^{b}R^{c}, -C(=O)R^{a}, -C(=O)OR^{b}, -C(=O)NR^{b}R^{c}, C₁-C₆ alkyl, or C₁-C₆ haloalkyl, and
contacting the compound of formula (III-a) or a salt thereof with a second organic solvent and a reducing agent to form a compound of formula (IV-a) or a salt thereof:

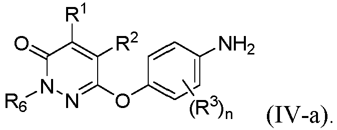

2. The method of claim 1, further comprising contacting the compound of formula (IV) or a salt thereof with R⁴CH₂C(O)N(R⁵)C(O)OCH₂CH₃ to form a compound of formula (V) or a salt thereof: 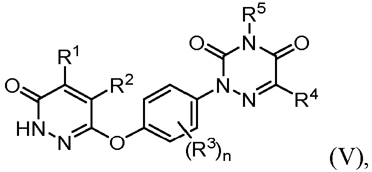 wherein:
R⁴ is hydrogen, deuterium, halogen, -CN, -OH, -OR^{a}, -SH, -SR^{a}, -S(=O)R^{a}, - S(=O)₂R^{a}, -NO₂, -NR^{b}R^{c}, -NHS(=O)₂R^{a}, -S(=O)₂NR^{b}R^{c}, -C(=O)R^{a}, -OC(=O)R^{a}, -C(=O)OR^{b}, -OC(=O)OR^{b}, -C(=O)NR^{b}R^{c}, -OC(=O)NRbR^{c}, -NR^{b}C(=O)NR^{b}R^{c}, -NR^{b}C(=O)R^{a}, - NR^{b}C(=O)OR^{b}, C₁-C₆ alkyl, C₁-C₆ deuteroalkyl, C₁-C₆ haloalkyl, C₁-C₆ hydroxyalkyl, C₁-C₆ aminoalkyl, C₂-C₆ alkenyl, C₂-C₆ alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, - OR^{a}, -NR^{b}R^{c}, -C(=O)R^{a}, -C(=O)OR^{b}, -C(=O)NR^{b}R^{c}, C₁-C₆ alkyl, or C₁-C₆ haloalkyl; and
R⁵ is hydrogen, deuterium, halogen, -CN, -OH, -OR^{a}, -S(=O)R^{a}, -S(=O)₂R^{a}, - S(=O)₂NR^{b}R^{c}, -C(=O)R^{a}, -OC(=O)R^{a}, -C(=O)OR^{b}, -C(=O)NR^{b}R^{c}, C₁-C₆ alkyl, C₁-C₆ deuteroalkyl, C₁-C₆ haloalkyl, C₁-C₆ hydroxyalkyl, C₁-C₆ aminoalkyl, C₂-C₆ alkenyl, C₂-C₆ alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OR^{a}, -NR^{b}R^{c}, -C(=O)R^{a}, - C(=O)OR^{b}, -C(=O)NR^{b}R^{c}, C₁-C₆ alkyl, or C₁-C₆ haloalkyl.

3. The method of claim 1, further comprising contacting the compound of formula (IV-a) or a salt thereof with R⁴CH₂C(O)N(R⁵)C(O)OCH₂CH₃ to form a compound of formula (V-a) or a salt thereof: 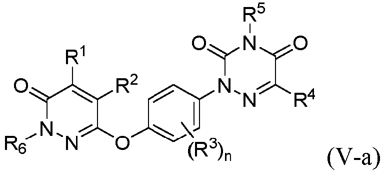 wherein:
R⁴ is hydrogen, deuterium, halogen, -CN, -OH, -OR^{a}, -SH, -SR^{a}, -S(=O)R^{a}, - S(=O)₂R^{a}, -NO₂, -NR^{b}R^{c}, -NHS(=O)₂R^{a}, -S(=O)₂NR^{b}R^{c}, -C(=O)R^{a}, -OC(=O)R^{a}, -C(=O)OR^{b}, -OC(=O)OR^{b}, -C(=O)NR^{b}R^{c}, -OC(=O)NRbR^{c}, -NR^{b}C(=O)NR^{b}R^{c}, -NR^{b}C(=O)R^{a}, - NR^{b}C(=O)OR^{b}, C₁-C₆ alkyl, C₁-C₆ deuteroalkyl, C₁-C₆ haloalkyl, C₁-C₆ hydroxyalkyl, C₁-C₆ aminoalkyl, C₂-C₆ alkenyl, C₂-C₆ alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, - OR^{a}, -NR^{b}R^{c}, -C(=O)R^{a}, -C(=O)OR^{b}, -C(=O)NR^{b}R^{c}, C₁-C₆ alkyl, or C₁-C₆ haloalkyl; and
R⁵ is hydrogen, halogen, -CN, -OH, -OR^{a}, -S(=O)R^{a}, -S(=O)₂R^{a}, -S(=O)₂NR^{b}R^{c}, - C(=O)R^{a}, -OC(=O)R^{a}, -C(=O)OR^{b}, -C(=O)NR^{b}R^{c}, C₁-C₆ alkyl, C₁-C₆ deuteroalkyl, C₁-C₆ haloalkyl, C₁-C₆ hydroxyalkyl, C₁-C₆ aminoalkyl, C₂-C₆ alkenyl, C₂-C₆ alkynyl, cycloalkyl, heterocycloalkyl, aryl, or heteroaryl; wherein each alkyl, alkenyl, alkynyl, cycloalkyl, heterocycloalkyl, aryl, and heteroaryl is independently optionally substituted with one or more oxo, deuterium, halogen, -CN, -OH, -OR^{a}, -NR^{b}R^{c}, -C(=O)R^{a}, -C(=O)OR^{b}, - C(=O)NR^{b}R^{c}, C₁-C₆ alkyl, or C₁-C₆ haloalkyl.

4. The method of claim 1, wherein X is F.

5. The method of claim 1, wherein R³ is Cl.

6. The method of claim 1, wherein the compound of formula (I) is compound 1-1, compound 2-1, compound 3-1, compound 4-1, or compound 5-1:

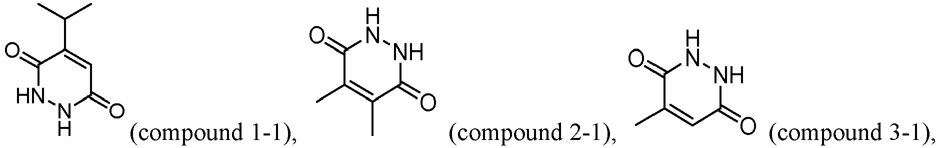

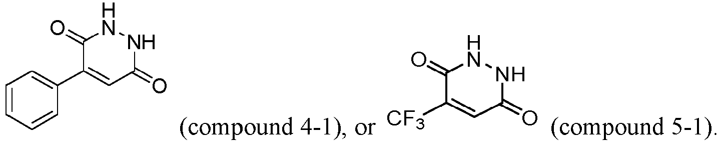

7. The method of claim 1, wherein the compound of formula (II) is compound 1-2:

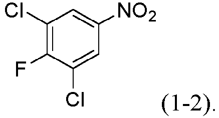

8. The method of claim 1, wherein the compound of formula (III) is compound 1-4, compound 2-2, compound 3-2, compound 3-3, compound 4-2, or compound 5-2:

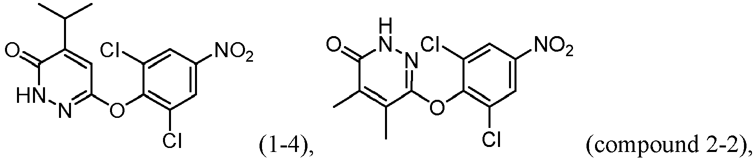

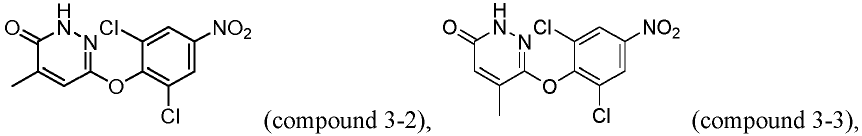

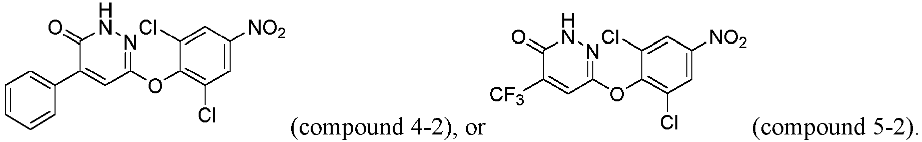

9. The method of claim 1, wherein the compound of formula (IV) or the salt thereof is Int. 7, compound 2-3, compound 3-4, compound 3-5, compound 4-3, compound 5-3, or compound 8-1: 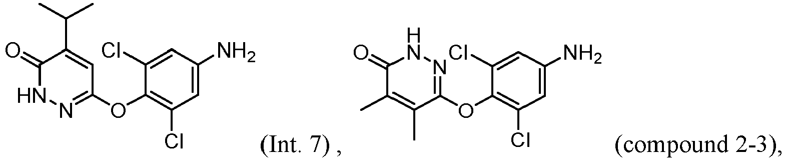 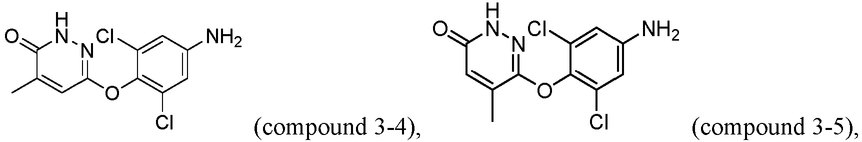 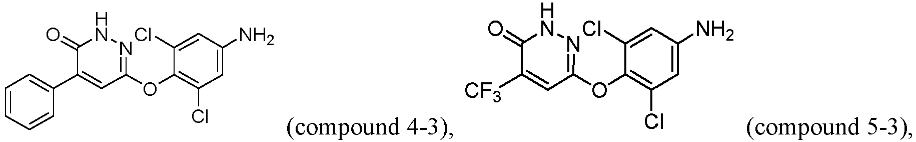 or

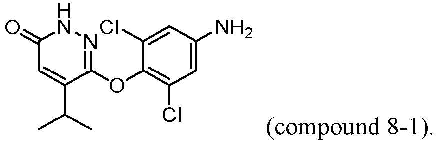

10. The method of claim 1, wherein:
(a) the first organic solvent comprises DMF, DMAC, DMSO, acetonitrile, THF, DCM, dioxane, or acetone; optionally, the first organic solvent comprises THF;
(b) the base comprises KHCO₃ or K₂CO₃;
(c) the reducing agent comprises H₂ gas and Pd/C, H₂ gas and Raney^{®} nickel, H₂ gas and platinum (IV) oxide, ferrous chloride, or stannous chloride; optionally, the reducing agent comprises H₂ gas and Pd/C; and/or
(d) second organic solvent comprises DMF, DMAC, DMSO, acetonitrile, THF, DCM, dioxane, or acetone.

11. The method of claim 1, wherein the contacting occurs at room temperature or above room temperature.

12. The method of claim 2, wherein the compound of formula (V) is 2-(3,5-dichloro-4-((5-isopropyl-6-oxo-1,6-dihydropyridazin-3-yl)oxy)phenyl)-3,5-dioxo-2,3,4,5-tetrahydro-1,2,4-triazine-6-carbonitrile (MGL-3196).

13. A compound having the structure of 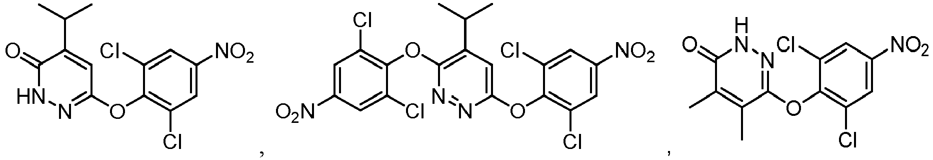 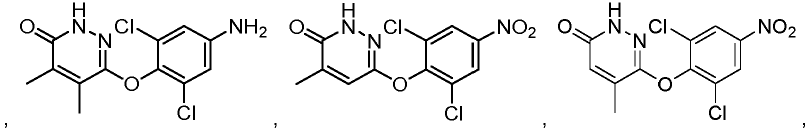 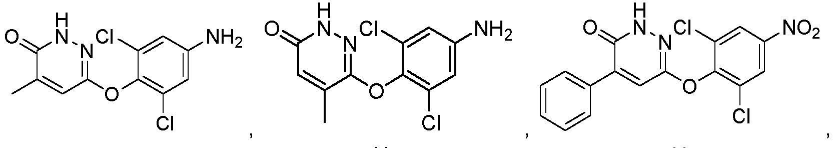 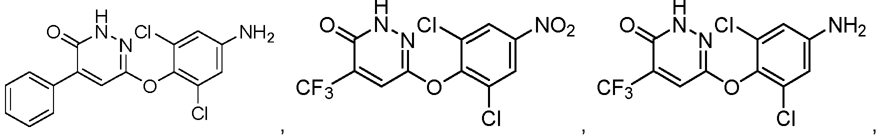 or

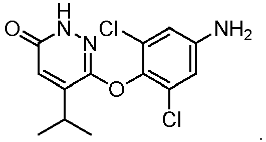

## Patentansprüche

1. Verfahren, umfassend das Inkontaktbringen einer Verbindung mit Formel (I), eines Tautomers oder eines Salzes davon: 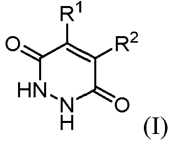 mit einer Verbindung der Formel (II) oder einem Salz davon: 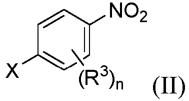 in einem ersten organischen Lösungsmittel in Gegenwart einer Base, um eine Verbindung der Formel (III) oder ein Salz davon zu bilden: 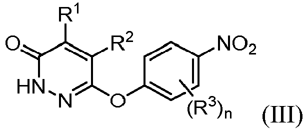 und
(A) Inkontaktbringen der Verbindung der Formel (III) oder eines Salzes davon mit einem zweiten organischen Lösungsmittel und einem Reduktionsmittel, um eine Verbindung der Formel (IV) oder ein Salz davon zu bilden: 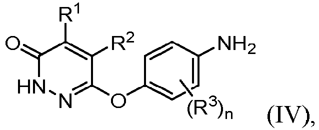 wobei:
R¹ und R² jeweils unabhängig Wasserstoff, Deuterium, Halogen, -CN, -OH, -OR^{a}, -SH, -SR^{a}, -S(=O)R^{a}, -S(=O)₂R^{a}, - NO₂, -NR^{b}R^{c}, -NHS(=O)₂R^{a}, -S(=O)₂NR^{b}R^{c}, -C(=O)R^{a}, -OC(=O)R^{a}, -C(=O)OR^{b}, -OC(=O)OR^{b}, -C(=O)NR^{b}R^{c}, -OC(=O)NR^{b}R^{c}, - NR^{b}C(=O)NR^{b}R^{c}, -NR^{b}C(=O)R^{a}, -NR^{b}C(=O)OR^{b}, C₁-C₆-Alkyl, C₁-C₆-Deuteroalkyl, C₁-C₆-Halogenalkyl, C₄-C₆-Hydroxyalkyl, C₁-C₆-Aminoalkyl, C₂-C₆-Alkenyl, C₂-C₆-Alkinyl, Cycloalkyl, Heterocycloalkyl, Aryl oder Heteroaryl sind; wobei jedes Alkyl, Alkenyl, Alkinyl, Cycloalkyl, Heterocycloalkyl, Aryl und Heteroaryl unabhängig gegebenenfalls mit einem oder mehreren Oxo, Deuterium, Halogen, -CN, -OH, -OR^{a}, -NR^{b}R^{c}, -C(=O)R^{a}, -C(=O)OR^{b}, - C(=O)NR^{b}R^{c}, C₁-C₆-Alkyl oder C₁-C₆-Halogenalkyl substituiert ist;
jedes R³ unabhängig Wasserstoff, Deuterium, Halogen, -CN, -OH, -OR^{a}, -SH, -SR^{a}, -S(=O)R^{a}, -S(=O)₂R^{a}, -NO₂, -NR^{b}R^{c}, - NHS(=O)₂R^{a}, -S(=O)₂NR^{b}R^{c}, -C(=O)R^{a}, -OC(=O)R^{a}, -C(=O)OR^{b}, - OC(=O)OR^{b}, -C(=O)NR^{b}R^{c}, -OC(=O)NR^{b}R^{c}, -NR^{b}C(=O)NR^{b}R^{c}, - NR^{b}C(=O)R^{a}, -NR^{b}C(=O)OR^{b}, C₁-C₆-Alkyl, C₁-C₆-Deuteroalkyl,
C₁-C₆-Halogenalkyl, C₁-C₆-Hydroxyalkyl, C₁-C₆-Aminoalkyl, C₂-C₆-Alkenyl, C₂-C₆-Alkinyl, Cycloalkyl, Heterocycloalkyl, Aryl oder Heteroaryl ist; wobei jedes Alkyl, Alkenyl, Alkinyl, Cycloalkyl, Heterocycloalkyl, Aryl und Heteroaryl unabhängig gegebenenfalls mit einem oder mehreren Oxo, Deuterium, Halogen, -CN, -OH, -OR^{a}, - NR^{b}R^{c}, -C(=O)R^{a}, -C(=O)OR^{b}, -C(=O)NR^{b}R^{c}, C₁-C₆-Alkyl oder C₁-C₆-Halogenalkyl substituiert ist;
jedes R^{a} unabhängig C₁-C₆-Alkyl, C₁-C₆-Deuteroalkyl, C₁-C₆-Halogenalkyl, C₁-C₆-Hydroxyalkyl, C₁-C₆-Aminoalkyl, C₂-C₆-Alkenyl, C₂-C₆-Alkinyl, Cycloalkyl, Heterocycloalkyl, Aryl oder Heteroaryl ist; wobei jedes Alkyl, Alkenyl, Alkinyl, Cycloalkyl, Heterocycloalkyl, Aryl und Heteroaryl unabhängig gegebenenfalls mit einem oder mehreren Oxo, Deuterium, Halogen, -CN-, -OH, -OCH₃, -NH₂, -C(=O)CH₃, -C(=O)OH, -C(=O)OCH₃, C₁-C₆-Alkyl oder C₁-C₆-Halogenalkyl substituiert ist;
jedes R^{b} unabhängig Wasserstoff, Deuterium, C₁-C₆-Alkyl, C₁-C₆-Deuteroalkyl, C₁-C₆-Halogenalkyl, C₁-C₆-Hydroxyalkyl, C₁-C₆-Aminoalkyl, C₂-C₆-Alkenyl, C₂-C₆-Alkinyl, Cycloalkyl, Heterocycloalkyl, Aryl oder Heteroaryl ist; wobei jedes Alkyl, Alkenyl, Alkinyl, Cycloalkyl, Heterocycloalkyl, Aryl und Heteroaryl unabhängig gegebenenfalls mit einem oder mehreren Oxo, Deuterium, Halogen, -CN-, -OH, -OCH₃, -NH₂, -C(=O)CH₃, - C(=O)OH, -C(=O)OCH₃, C₁-C₆-Alkyl oder C₁-C₆-Halogenalkyl substituiert ist;
jedes R^{c} unabhängig Wasserstoff, Deuterium, C₁-C₆-Alkyl, C₁-C₆-Deuteroalkyl, C₁-C₆-Halogenalkyl, C₁-C₆-Hydroxyalkyl, C₁-C₆-Aminoalkyl, C₂-C₆-Alkenyl, C₂-C₆-Alkinyl, Cycloalkyl, Heterocycloalkyl, Aryl oder Heteroaryl ist; wobei jedes Alkyl, Alkenyl, Alkinyl, Cycloalkyl, Heterocycloalkyl, Aryl und Heteroaryl unabhängig gegebenenfalls mit einem oder mehreren Oxo, Deuterium, Halogen, -CN-, -OH, -OCH₃, -NH₂, -C(=O)CH₃, - C(=O)OH, -C(=O)OCH₃, C₁-C₆-Alkyl oder C₁-C₆-Halogenalkyl substituiert ist;
X Halogen ist; und
n 0, 1, 2, 3 oder 4 ist;
(B) Inkontaktbringen der Verbindung der Formel (III) oder eines Salzes davon mit R⁶X, um eine Verbindung der Formel (III-A) oder ein Salz davon zu bilden: 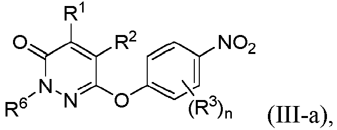 wobei:
R⁶-CN, -OH, -OR^{a}, -S(=O)R^{a}, -S(=O)₂R^{a}, -S(=O)₂NR^{b}R^{c}, - C(=O)R^{a}, -OC(=O)R^{a}, -C(=O)OR^{b}, -C(=O)NR^{b}R^{c}, C₁-C₆-Alkyl, C₁-C₆-Deuteroalkyl, C₁-C₆-Halogenalkyl, C₁-C₆ - Hydroxyalkyl, C₁-C₆-Aminoalkyl, C₂-C₆-Alkenyl, C₂-C₆-Alkinyl, Cycloalkyl, Heterocycloalkyl, Aryl oder Heteroaryl ist; wobei jedes Alkyl, Alkenyl, Alkinyl, Cycloalkyl, Heterocycloalkyl, Aryl und Heteroaryl unabhängig gegebenenfalls mit einem oder mehreren Oxo, Deuterium, Halogen, -CN, -OH, -OR^{a}, -NR^{b}R^{c}, -C(=O)R^{a}, - C(=O)OR^{b}, -C(=O)NR^{b}R^{c}, C₁-C₆-Alkyl oder C₁-C₆-Halogenalkyl substituiert ist, und
Inkontaktbringen der Verbindung der Formel (III-a) oder eines Salzes davon mit einem zweiten organischen Lösungsmittel und einem Reduktionsmittel, um eine Verbindung der Formel (IV-a) oder ein Salz davon zu bilden:

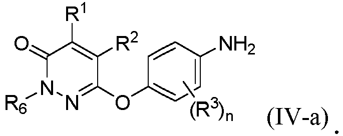

2. Verfahren nach Anspruch 1, ferner umfassend das Inkontaktbringen der Verbindung der Formel (IV) oder eines Salzes davon mit R⁴CH₂C(O)N(R⁵)C(O)OCH₂CH₃, um eine Verbindung der Formel (V) oder ein Salz davon zu bilden: 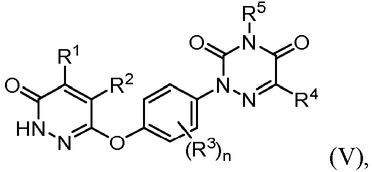 wobei:
R⁴ Wasserstoff, Deuterium, Halogen, -CN, -OH, -OR^{a}, -SH, -SR^{a}, -S(=O)R^{a}, -S(=O)₂R^{a}, -NO₂, -NR^{b}R^{c}, -NHS(=O)₂R^{a}, - S(=O)₂NR^{b}R^{c}, -C(=O)R^{a}, -OC(=O)R^{a}, -C(=O)OR^{b}, -OC(=O)OR^{b}, - C(=O)NR^{b}R^{c}, -OC(=O)NR^{b}R^{c}, -NR^{b}C(=O)NR^{b}R^{c}, -NR^{b}C(=O)R^{a}, - NR^{b}C(=O)OR^{b}, C₁-C₆-Alkyl, C₁-C₆-Deuteroalkyl, C₁-C₆-Halogenalkyl, C₁-C₆-Hydroxyalkyl, C₁-C₆-Aminoalkyl, C₂-C₆-Alkenyl, C₂-C₆-Alkinyl, Cycloalkyl, Heterocycloalkyl, Aryl oder Heteroaryl ist; wobei jedes Alkyl, Alkenyl, Alkinyl, Cycloalkyl, Heterocycloalkyl, Aryl und Heteroaryl unabhängig gegebenenfalls mit einem oder mehreren Oxo, Deuterium, Halogen, -CN, -OH, -OR^{a}, -NR^{b}R^{c}, -C(=O)R^{a}, -C(=O)OR^{b}, -C(=O)NR^{b}R^{c}, C₁-C₆-Alkyl oder C₁-C₆-Halogenalkyl substituiert ist; und
R⁵ Wasserstoff, Deuterium, Halogen, -CN, -OH, -OR^{a}, - S(=O)R^{a}, -S(=O)₂R^{a}, -S(=O)₂NR^{b}R^{c}, -C(=O)R^{a}, -OC(=O)R^{a}, - C(=O)OR^{b}, -C(=O)NR^{b}R^{c}, C₁-C₆-Alkyl, C₁-C₆-Deuteroalkyl, C₁-C₆-Halogenalkyl, C₁-C₆ -Hydroxyalkyl, C₁-C₆-Aminoalkyl, C₂-C₆-Alkenyl, C₂-C₆-Alkinyl, Cycloalkyl, Heterocycloalkyl, Aryl oder Heteroaryl ist; wobei jedes Alkyl, Alkenyl, Alkinyl, Cycloalkyl, Heterocycloalkyl, Aryl und Heteroaryl unabhängig gegebenenfalls mit einem oder mehreren Oxo, Deuterium, Halogen, -CN, -OH, -OR^{a}, - NR^{b}R^{c}, -C(=O)R^{a}, -C(=O)OR^{b}, -C(=O)NR^{b}R^{c}, C₁-C₆-Alkyl oder C₁-C₆-Halogenalkyl substituiert ist.

3. Verfahren nach Anspruch 1, ferner umfassend das Inkontaktbringen der Verbindung der Formel (IV-a) oder eines Salzes davon mit R⁴CH₂C(O)N(R⁵)C(O)OCH₂CH₃, um eine Verbindung der Formel (V-a) oder ein Salz davon zu bilden: 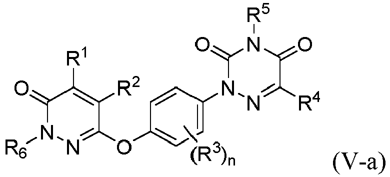 wobei:
R⁴ Wasserstoff, Deuterium, Halogen, -CN, -OH, -OR^{a}, -SH, -SR^{a}, -S(=O)R^{a}, -S(=O)₂R^{a}, -NO₂, -NR^{b}R^{c}, -NHS (=O)₂R^{a}, - S(=O)₂NR^{b}R^{c}, -C(=O)R^{a}, -OC(=O)R^{a}, -C(=O)OR^{b}, -OC(=O)OR^{b}, - C(=O)NR^{b}R^{c}, -OC(=O)NR^{b}R^{c}, -NR^{b}C(=O)NR^{b}R^{c}, -NR^{b}C(=O)R^{a}, - NR^{b}C(=O)OR^{b}, C₁-C₆-Alkyl, C₁-C₆-Deuteroalkyl, C₁-C₆-Halogenalkyl, C₁-C₆-Hydroxyalkyl, C₁-C₆-Aminoalkyl, C₂-C₆-Alkenyl, C₂-C₆-Alkinyl, Cycloalkyl, Heterocycloalkyl, Aryl oder Heteroaryl ist; wobei jedes Alkyl, Alkenyl, Alkinyl, Cycloalkyl, Heterocycloalkyl, Aryl und Heteroaryl unabhängig gegebenenfalls mit einem oder mehreren Oxo, Deuterium, Halogen, -CN, -OH, -OR^{a}, -NR^{b}R^{c}, -C(=O)R^{a}, -C(=O)OR^{b}, -C(=O)NR^{b}R^{c}, C₁-C₆-Alkyl oder C₁-C₆-Halogenalkyl substituiert ist; und
R⁵ Wasserstoff, Halogen, -CN, -OH, -OR^{a}, -S(=O)R^{a}, - S(=O)₂R^{a}, -S(=O)₂NR^{b}R^{c}, -C(=O)R^{a}, -OC(=O)R^{a}, -C(=O)OR^{b}, - C(=O)NR^{b}R^{c}, C₁-C₆-Alkyl, C₁-C₆-Deuteroalkyl, C₁-C₆-Halogenalkyl, C₁-C₆ -Hydroxyalkyl, C₁-C₆-Aminoalkyl, C₂-C₆-Alkenyl, C₂-C₆ Alkinyl, Cycloalkyl, Heterocycloalkyl, Aryl oder Heteroaryl ist; wobei jedes Alkyl, Alkenyl, Alkinyl, Cycloalkyl, Heterocycloalkyl, Aryl und Heteroaryl unabhängig gegebenenfalls mit einem oder mehreren Oxo, Deuterium, Halogen, -CN, -OH, -OR^{a}, -NR^{b}R^{c}, -C(=O)R^{a}, -C(=O)OR^{b}, -C(=O)NR^{b}R^{c}, C₁-C₆-Alkyl oder C₁-C₆-Halogenalkyl substituiert ist.

4. Verfahren nach Anspruch 1, wobei X F ist.

5. Verfahren nach Anspruch 1, wobei R³ Cl ist.

6. Verfahren nach Anspruch 1, wobei die Verbindung der Formel (I) Verbindung 1-1, Verbindung 2-1, Verbindung 3-1, Verbindung 4-1 oder Verbindung 5-1 ist:

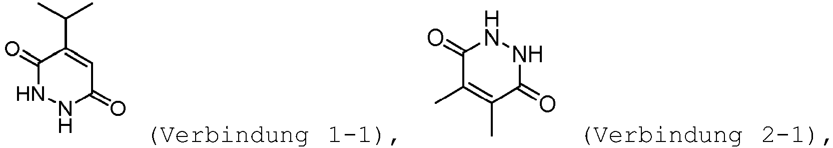

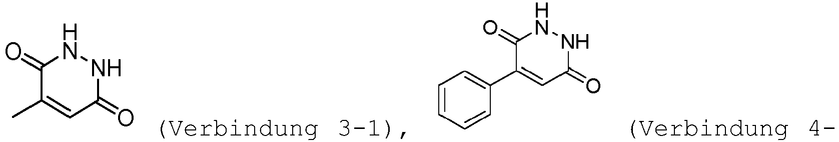

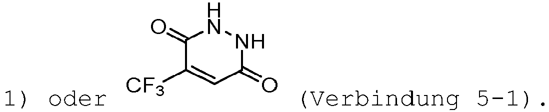

7. Verfahren nach Anspruch 1, wobei die Verbindung der Formel (II) Verbindung 1-2 ist:

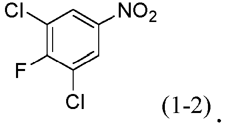

8. Verfahren nach Anspruch 1, wobei die Verbindung der Formel (III) Verbindung 1-4, Verbindung 2-2, Verbindung 3-2, Verbindung 3-3, Verbindung 4-2 oder Verbindung 5-2 ist:

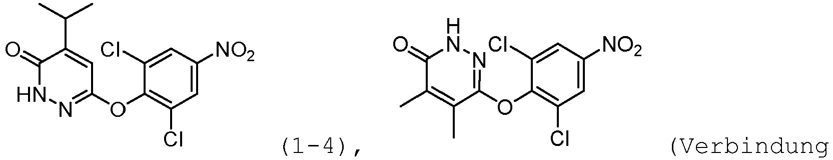

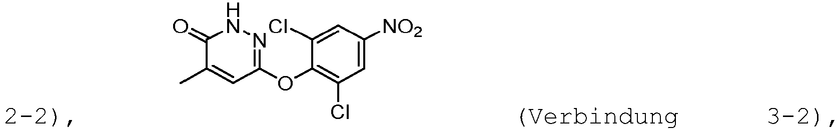

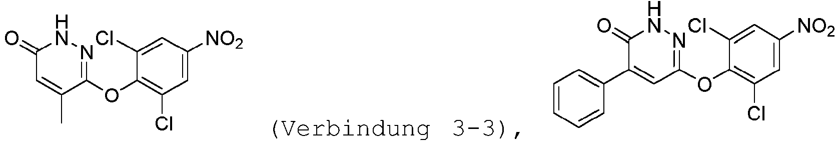

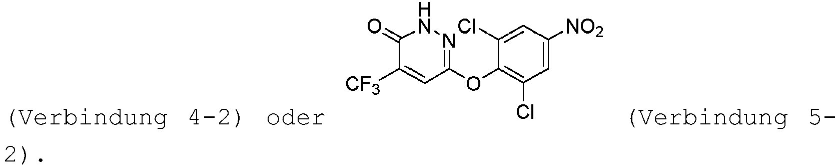

9. Verfahren nach Anspruch 1, wobei die Verbindung der Formel (IV) oder das Salz davon Zwisch. 7, Verbindung 2-3, Verbindung 3-4, Verbindung 3-5, Verbindung 4-3, Verbindung 5-3 oder Verbindung 8-1 ist: 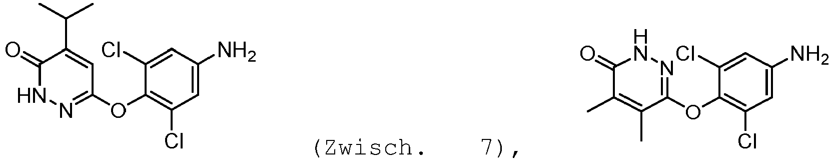 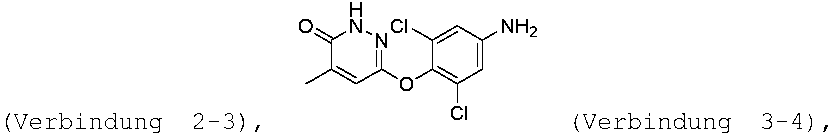 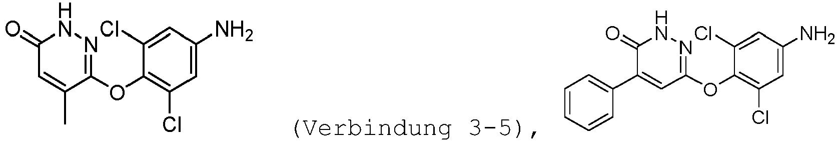 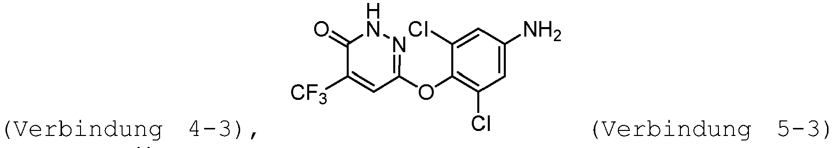 oder

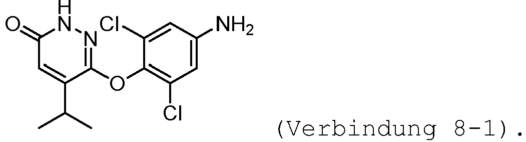

10. Verfahren nach Anspruch 1, wobei:
(a) das erste organische Lösungsmittel DMF, DMAC, DMSO, Acetonitril, THF, DCM, Dioxan oder Aceton umfasst; das erste organische Lösungsmittel gegebenenfalls THF umfasst;
(b) die Base KHCO₃ oder K₂CO₃ umfasst;
(c) das Reduktionsmittel H₂-Gas und Pd/C, H₂-Gas und Raney^{®}-Nickel, H₂-Gas und Platin(IV)-Oxid, Eisen(II)-chlorid oder Zinnchlorid umfasst; das Reduktionsmittel gegebenenfalls H₂-Gas und Pd/C umfasst; und/oder
(d) das zweite organische Lösungsmittel DMF, DMAC, DMSO, Acetonitril, THF, DCM, Dioxan oder Aceton umfasst.

11. Verfahren nach Anspruch 1, wobei das Inkontaktbringen bei Raumtemperatur oder über Raumtemperatur erfolgt.

12. Verfahren nach Anspruch 2, wobei die Verbindung der Formel (V) 2-(3,5-Dichlor-4-((5-isopropyl-6-oxo-1,6-dihydropyridazin-3-yl)oxy)phenyl)-3,5-dioxo-2,3,4,5-tetrahydro-1,2,4-triazin-6-carbonitril (MGL-3196) ist.

13. Verbindung mit der Struktur von 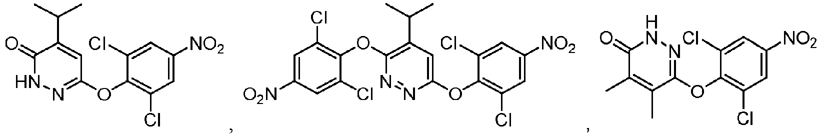 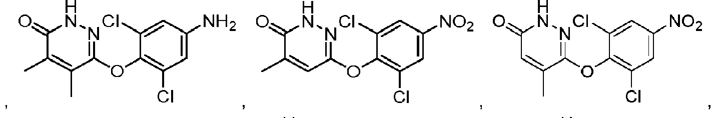 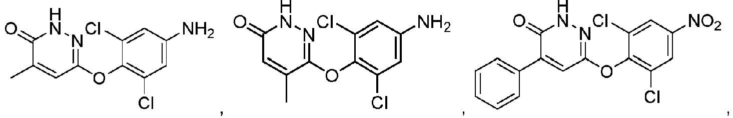 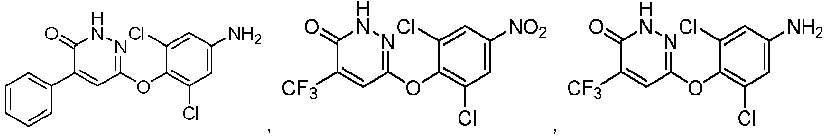 oder

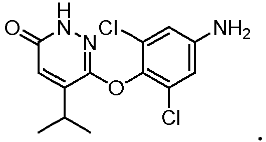

## Revendications

1. Procédé comprenant la mise en contact d'un composé de formule (I), d'une forme tautomère ou d'un sel de celui-ci : 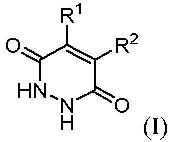 avec un composé de formule (II) ou un sel de celui-ci : 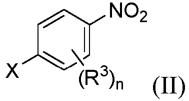 dans un premier solvant organique en présence d'une base, pour former un composé de formule (III) ou un sel de celui-ci : 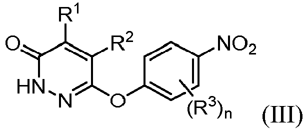 et
(A) la mise en contact du composé de formule (III) ou d'un sel de celui-ci avec un deuxième solvant organique et un agent réducteur pour former un composé de formule (IV) ou un sel de celui-ci : 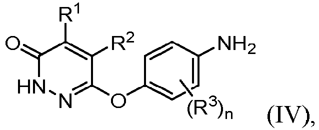
R¹ et R² étant chacun indépendamment hydrogène, deutérium, halogène, -CN, -OH, -OR^{a}, -SH, -SR^{a}, -S(=O)R^{a}, -S(=O)₂R^{a}, -NO₂, -NR^{b}R^{c}, -NHS(=O)₂R^{a}, -S(=O)₂NR^{b}R^{c}, -C(=O)R^{a}, - OC(=O)R^{a}, -C(=O)OR^{b}, -OC(=O)OR^{b}, -C(=O)NR^{b}R^{c}, -OC(=O)NR^{b}R^{c}, -NR^{b}C(=O)NR^{b}R^{c}, -NR^{b}C(=O)R^{a}, -NR^{b}C(=O)OR^{b}, C₁-C₆ alkyle, C₁-C₆ deutéroalkyle, C₁-C₆ halogénoalkyle, C₄-C₆ hydroxyalkyle, C₁-C₆ aminoalkyle, C₂-C₆ alcényle, C₂-C₆ alcynyle, cycloalkyle, hétérocycloalkyle, aryle, ou hétéroaryle ; chaque alkyle, alcényle, alcynyle, cycloalkyle, hétérocycloalkyle, aryle, et hétéroaryle étant indépendamment éventuellement substitué par un ou plusieurs oxo, deutérium, halogène, -CN, -OH, -OR^{a}, - NR^{b}R^{c}, -C(=O)R^{a}, -C(=O)OR^{b}, -C(=O)NR^{b}R^{c}, C₁-C₆ alkyle, ou C₁-C₆ halogénoalkyle ;
chaque R³ étant indépendamment hydrogène, deutérium, halogène, -CN, -OH, -OR^{a}, -SH, -SR^{a}, -S(=O)R^{a}, -S(=O)₂R^{a}, -NO₂, -NR^{b}R^{c}, -NHS(=O)₂R^{a}, -S(=O)₂NR^{b}R^{c}, -C(=O)R^{a}, - OC(=O)R^{a}, -C(=O)OR^{b}, -OC(=O)OR^{b}, -C(=O)NR^{b}R^{c}, -OC(=O)NR^{b}R^{c}, -NR^{b}C(=O)NR^{b}R^{c}, -NR^{b}C(=O)R^{a}, -NR^{b}C(=O)OR^{b}, C₁-C₆ alkyle, C₁-C₆ deutéroalkyle, C₁-C₆ halogénoalkyle, C₁-C₆ hydroxyalkyle, C₁-C₆ aminoalkyle, C₂-C₆ alcényle, C₂-C₆ alcynyle, cycloalkyle, hétérocycloalkyle, aryle, ou hétéroaryle ; chaque alkyle, alcényle, alcynyle, cycloalkyle, hétérocycloalkyle, aryle, et hétéroaryle étant indépendamment éventuellement substitué par un ou plusieurs oxo, deutérium, halogène, -CN, -OH, -OR^{a}, - NR^{b}R^{c}, -C(=O)R^{a}, -C(=O)OR^{b}, -C(=O)NR^{b}R^{c}, C₁-C₆ alkyle, ou C₁-C₆ halogénoalkyle ;
chaque R^{a} étant indépendamment C₁-C₆ alkyle, C₁-C₆ deutéroalkyle, C₁-C₆ halogénoalkyle, C₁-C₆ hydroxyalkyle, C₁-C₆ aminoalkyle, C₂-C₆ alcényle, C₂-C₆ alcynyle, cycloalkyle, hétérocycloalkyle, aryle, ou hétéroaryle ; chaque alkyle, alcényle, alcynyle, cycloalkyle, hétérocycloalkyle, aryle, et hétéroaryle étant indépendamment éventuellement substitué par un ou plusieurs oxo, deutérium, halogène, -CN, -OH, -OCH₃, - NH₂, -C(=O)CH₃, -C(=O)OH, -C(=O)OCH₃, C₁-C₆alkyle, ou C₁-C₆ halogénoalkyle ;
chaque R^{b} étant indépendamment hydrogène, deutérium, C₁-C₆ alkyle, C₁-C₆ deutéroalkyle, C₁-C₆ halogénoalkyle, C₁-C₆ hydroxyalkyle, C₁-C₆ aminoalkyle, C₂-C₆ alcényle, C₂-C₆ alcynyle, cycloalkyle, hétérocycloalkyle, aryle, ou hétéroaryle ; chaque alkyle, alcényle, alcynyle, cycloalkyle, hétérocycloalkyle, aryle, et hétéroaryle étant indépendamment éventuellement substitué par un ou plusieurs oxo, deutérium, halogène, -CN, -OH, -OCH₃, - NH₂, -C(=O)CH₃, -C(=O)OH, -C(=O)OCH₃, C₁-C₆ alkyle, ou C₁-C₆ halogénoalkyle ;
chaque R^{c} étant indépendamment hydrogène, deutérium, C₁-C₆ alkyle, C₁-C₆ deutéroalkyle, C₁-C₆ halogénoalkyle, C₁-C₆ hydroxyalkyle, C₁-C₆ aminoalkyle, C₂-C₆ alcényle, C₂-C₆ alcynyle, cycloalkyle, hétérocycloalkyle, aryle, ou hétéroaryle ; chaque alkyle, alcényle, alcynyle, cycloalkyle, hétérocycloalkyle, aryle, et hétéroaryle étant indépendamment éventuellement substitué par un ou plusieurs oxo, deutérium, halogène, -CN, -OH, -OCH₃, - NH₂, -C(=O)CH₃, -C(=O)OH, -C(=O)OCH₃, C₁-C₆ alkyle, ou C₁-C₆ halogénoalkyle ;
X étant halogène ; et
n étant 0, 1, 2, 3, ou 4, ou
(B) la mise en contact du composé de formule (III) ou d'un sel de celui-ci avec R⁶X pour former un composé de formule (III-a) ou un sel de celui-ci : 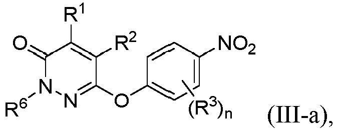
R⁶ étant -CN, -OH, -OR^{a}, -S(=O)R^{a}, -S(=O)₂R^{a}, -S(=O)₂NR^{b}R^{c}, -C(=O)R^{a}, -OC(=O)R^{a}, -C(=O)OR^{b}, -C(=O)NR^{b}R^{c}, C₁-C₆ alkyle, C₁-C₆ deutéroalkyle, C₁-C₆ halogénoalkyle, C₁-C₆ hydroxyalkyle, C₁-C₆ aminoalkyle, C₂-C₆ alcényle, C₂-C₆ alcynyle, cycloalkyle, hétérocycloalkyle, aryle, ou hétéroaryle ; chaque alkyle, alcényle, alcynyle, cycloalkyle, hétérocycloalkyle, aryle, et hétéroaryle étant indépendamment éventuellement substitué par un ou plusieurs oxo, deutérium, halogène, -CN, -OH, -OR^{a}, - NR^{b}R^{c}, -C(=O)R^{a}, -C(=O)OR^{b}, -C(=O)NR^{b}R^{c}, C₁-C₆ alkyle, ou C₁-C₆ halogénoalkyle, et
la mise en contact du composé de formule (III-a) ou d'un sel de celui-ci avec un deuxième solvant organique et un agent réducteur pour former un composé de formule (IV-a) ou un sel de celui-ci :

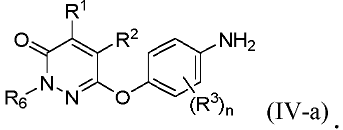

2. Procédé selon la revendication 1, comprenant en outre la mise en contact du composé de formule (IV) ou d'un sel de celui-ci avec R⁴CH₂C(O)N(R⁵)C(O)OCH₂CH₃ pour former un composé de formule (V) ou un sel de celui-ci : 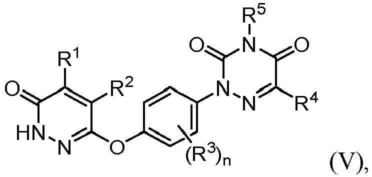
R⁴ étant hydrogène, deutérium, halogène, -CN, -OH, -OR^{a}, -SH, -SR^{a}, -S(=O)R^{a}, -S(=O)₂R^{a}, -NO₂, -NR^{b}R^{c}, -NHS(=O)₂R^{a}, -S(=O)₂NR^{b}R^{c}, -C(=O)R^{a}, -OC(=O)R^{a}, -C(=O)OR^{b}, -OC(=O)OR^{b}, -C(=O)NR^{b}R^{c}, -OC(=O)NR^{b}R^{c}, -NR^{b}C(=O)NR^{b}R^{c}, -NR^{b}C(=O)R^{a}, - NR^{b}C(=O)OR^{b}, C₁-C₆ alkyle, C₁-C₆ deutéroalkyle, C₁-C₆ halogénoalkyle, C₁-C₆ hydroxyalkyle, C₁-C₆ aminoalkyle,
C₂-C₆ alcényle, C₂-C₆ alcynyle, cycloalkyle, hétérocycloalkyle, aryle, ou hétéroaryle ; chaque alkyle, alcényle, alcynyle, cycloalkyle, hétérocycloalkyle, aryle, et hétéroaryle étant indépendamment éventuellement substitué par un ou plusieurs oxo, deutérium, halogène, -CN, -OH, -OR^{a}, - NR^{b}R^{c}, -C(=O)R^{a}, -C(=O)OR^{b}, -C(=O)NR^{b}R^{c}, C₁-C₆ alkyle, ou C₁-C₆ halogénoalkyle ; et
R⁵ étant hydrogène, deutérium, halogène, -CN, -OH, -OR^{a}, -S(=O)R^{a}, -S(=O)₂R^{a}, -S(=O)₂NR^{b}R^{c}, -C(=O)R^{a}, -OC(=O)R^{a}, - C(=O)OR^{b}, -C(=O)NR^{b}R^{c}, C₁-C₆ alkyle, C₁-C₆ deutéroalkyle, C₁-C₆ halogénoalkyle, C₁-C₆ hydroxyalkyle, C₁-C₆ aminoalkyle, C₂-C₆ alcényle, C₂-C₆ alcynyle, cycloalkyle, hétérocycloalkyle, aryle, ou hétéroaryle ; chaque alkyle, alcényle, alcynyle, cycloalkyle, hétérocycloalkyle, aryle, et hétéroaryle étant indépendamment éventuellement substitué par un ou plusieurs oxo, deutérium, halogène, -CN, -OH, -OR^{a}, - NR^{b}R^{c}, -C(=O)R^{a}, -C(=O)OR^{b}, -C(=O)NR^{b}R^{c}, C₁-C₆ alkyle, ou C₁-C₆ halogénoalkyle.

3. Procédé selon la revendication 1, comprenant en outre la mise en contact du composé de formule (IV-a) ou d'un sel de celui-ci avec R⁴CH₂C(O)N(R⁵)C(O)OCH₂CH₃ pour former un composé de formule (V-a) ou un sel de celui-ci : 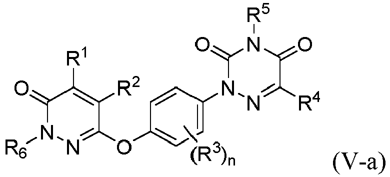
R⁴ étant hydrogène, deutérium, halogène, -CN, -OH, -OR^{a}, -SH, -SR^{a}, -S(=O)R^{a}, -S(=O)₂R^{a}, -NO₂, -NR^{b}R^{c}, -NHS(=O)₂R^{a}, -S(=O)₂NR^{b}R^{c}, -C(=O)R^{a}, -OC(=O)R^{a}, -C(=O)OR^{b}, -OC (=O) OR^{b}, -C(=O)NR^{b}R^{c}, -OC(=O)NR^{b}R^{c}, -NR^{b}C(=O)NR^{b}R^{c}, -NR^{b}C(=O)R^{a}, - NR^{b}C(=O)OR^{b}, C₁-C₆ alkyle, C₁-C₆ deutéroalkyle, C₁-C₆ halogénoalkyle, C₁-C₆ hydroxyalkyle, C₁-C₆ aminoalkyle, C₂-C₆ alcényle, C₂-C₆ alcynyle, cycloalkyle, hétérocycloalkyle, aryle, ou hétéroaryle ; chaque alkyle, alcényle, alcynyle, cycloalkyle, hétérocycloalkyle, aryle, et hétéroaryle étant indépendamment éventuellement substitué par un ou plusieurs oxo, deutérium, halogène, -CN, -OH, -OR^{a}, - NR^{b}R^{c}, -C(=O)R^{a}, -C(=O)OR^{b}, -C(=O)NR^{b}R^{c}, C₁-C₆ alkyle, ou C₁-C₆ halogénoalkyle ; et
R⁵ étant hydrogène, halogène, -CN, -OH, -OR^{a}, -S(=O)R^{a}, - S(=O)₂R^{a}, -S(=O)₂NR^{b}R^{c}, -C(=O)R^{a}, -OC(=O)R^{a}, -C(=O)OR^{b}, - C(=O)NR^{b}R^{c}, C₁-C₆ alkyle, C₁-C₆ deutéroalkyle, C₁-C₆ halogénoalkyle, C₁-C₆ hydroxyalkyle, C₁-C₆ aminoalkyle, C₂-C₆ alcényle, C₂-C₆ alcynyle, cycloalkyle, hétérocycloalkyle, aryle, ou hétéroaryle ; chaque alkyle, alcényle, alcynyle, cycloalkyle, hétérocycloalkyle, aryle, et hétéroaryle étant indépendamment éventuellement substitué par un ou plusieurs oxo, deutérium, halogène, -CN, -OH, -OR^{a}, - NR^{b}R^{c}, -C(=O)R^{a}, -C(=O)OR^{b}, -C(=O)NR^{b}R^{c}, C₁-C₆ alkyle, ou C₁-C₆ halogénoalkyle.

4. Procédé selon la revendication 1, X étant F.

5. Procédé selon la revendication 1, R³ étant Cl.

6. Procédé selon la revendication 1, le composé de formule (I) étant le composé 1-1, composé 2-1, composé 3-1, composé 4-1, ou composé 5-1 : 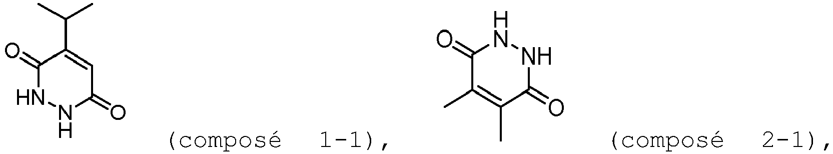 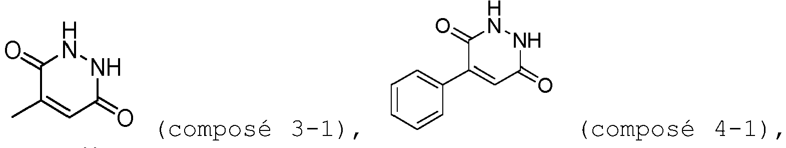 ou

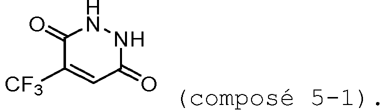

7. Procédé selon la revendication 1, le composé de formule (II) étant le composé 1-2 :

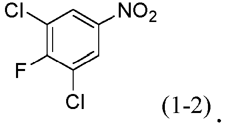

8. Procédé selon la revendication 1, le composé de formule (III) étant le composé 1-4, composé 2-2, composé 3-2, composé 3-3, composé 4-2, ou composé 5-2 : 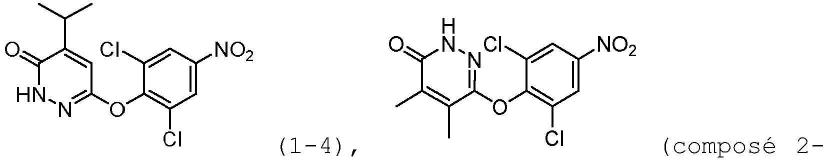 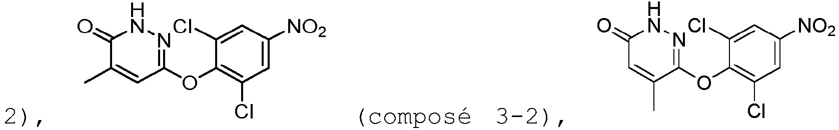 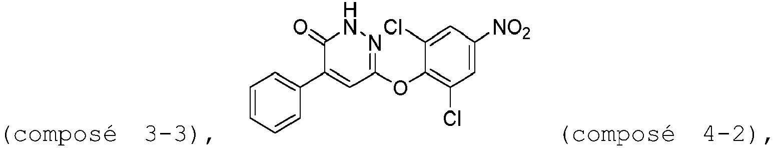 ou

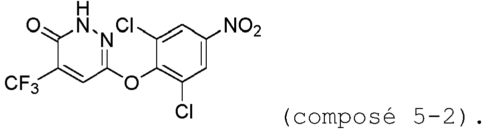

9. Procédé selon la revendication 1, le composé de formule (IV) ou le sel de celui-ci étant Int. 7, composé 2-3, composé 3-4, composé 3-5, composé 4-3, composé 5-3, ou composé 8-1 :

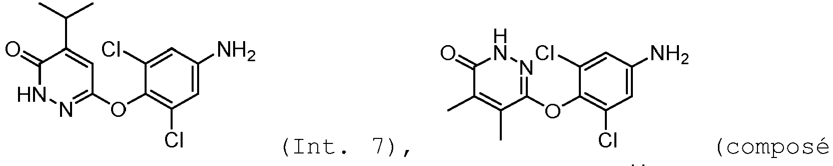

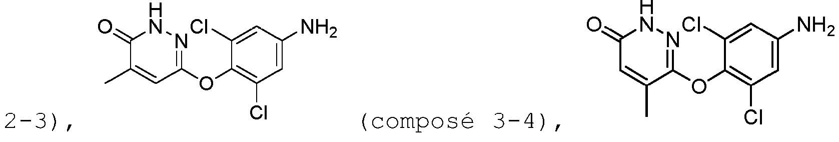

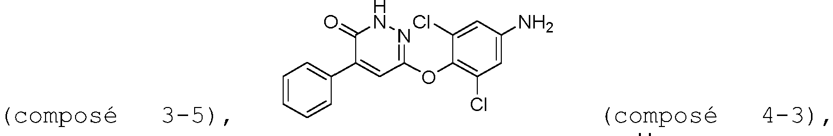

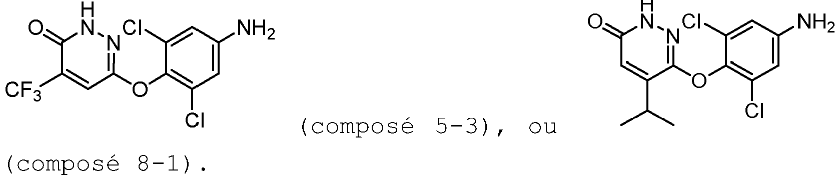

10. Procédé selon la revendication 1, dans lequel :
(a) le premier solvant organique comprend du DMF, du DMAC, du DMSO, de l'acétonitrile, du THF, du DCM, du dioxane, ou de l'acétone, éventuellement le premier solvant organique comprend du THF ;
(b) la base comprend KHCO₃ ou K₂CO₃ ;
(c) l'agent réducteur comprend du gaz de H₂ et Pd/C, du gaz de H₂ et du nickel de Raney^{®}, du gaz de H₂ et de l'oxyde de platine (IV), du chlorure ferreux ou du chlorure stanneux ; éventuellement, l'agent réducteur comprend du gaz de H₂ et Pd/C ; et/ou
(d) le deuxième solvant organique comprend du DMF, du DMAC, du DMSO, de l'acétonitrile, du THF, du DCM, du dioxane, ou de l'acétone.

11. Procédé selon la revendication 1, dans lequel la mise en contact se fait à température ambiante ou au-dessus de la température ambiante.

12. Procédé selon la revendication 2, dans lequel le composé de formule (V) est le 2-(3,5-dichloro-4-((5-isopropyl-6-oxo-1,6-dihydropyridazin-3-yl)oxy)phényl)-3,5-dioxo-2,3,4,5-tétrahydro-1,2,4-triazine-6-carbonitrile (MGL-3196).

13. Composé possédant la structure de 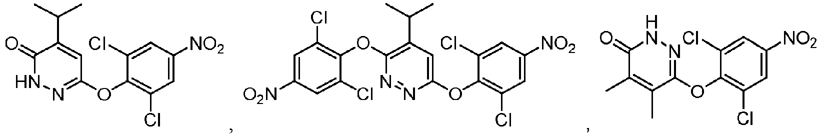 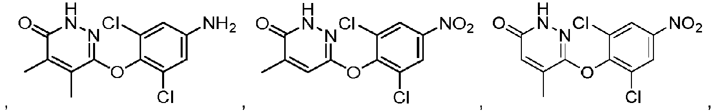 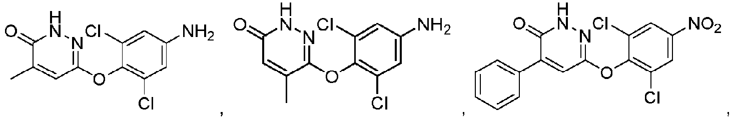 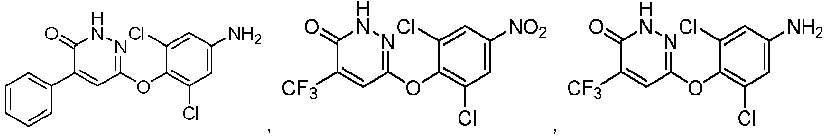 , ou
